# EUROPÄISCHE PATENTSCHRIFT

(11) **EP 1 901 695 B1**
(45) Veröffentlichungstag und Bekanntmachung des Hinweises auf die Patenterteilung: **18.04.2018**
(21) Anmeldenummer: 06762029.4
(22) Anmeldetag: 13.06.2006
(51) Int. Cl.: A61J 1/00

(54) **HERSTELLUNG KOLLOIDALISIERTER WIRKSTOFF- BEZIEHUNGSWEISE VITALSTOFFSPEZIES DURCH NANOSKALISCHE REAKTIVDESORPTION**
PRODUCTION OF COLLOIDAL ACTIVE SUBSTANCE OR VITAL SUBSTANCE SPECIES BY NANOSCALAR REACTIVE DESORPTION
PREPARATION D'ESPECES DE PRINCIPES ACTIFS COLLOIDES OU DE SUBSTANCES VITALES, PAR DESORPTION REACTIVE NANOSCALAIRE

(30) Priorität: 13.06.2005 DE 102005027333
(43) Veröffentlichungstag der Anmeldung: 26.03.2008
(73) Patentinhaber: Stephan, Katrin, 90556 Cadolzburg (DE)
(72) Erfinder: NOACK, Andreas, 55130 Mainz-Laubenheim (DE)
(74) Vertreter: Diehl & Partner GbR
(86) Internationale Anmeldenummer: PCT/EP2006/005669
(87) Internationale Veröffentlichungsnummer: WO 2006/133901

(56) Entgegenhaltungen:
- WO-A-2005/030111
- WO-A-2005/051104
- WO-A2-2005/084462
- US-A- 4 117 116
- US-A1- 2004 223 801

## Beschreibung

Die Erfindung betrifft ein Verfahren zur Solubilisierung von Wirkstoffen, schwerlöslichen Vitalstoffspezies sowie zur Herstellung ganzheitlicher bioverfügbarer kolloidalisierter Vitalstoffkonzentraten und -lösungen auf Basis lagerfähiger Einzelkomponenten mittels der nanoskalischen Reaktivdesorption, Varianten einer Vorrichtung zu deren Konfektionierung sowie die Verwendung des erzielten Endprodukts.

Unser Wissen zum Thema Vitalstoffe, die unseren Stoffwechsel und unsere Gesundheit positiv beeinflussen, wird immer umfangreicher. Wie im Fall der sekundären Pflanzeninhaltsstoffe (SPS) ist erst heute bekannt, dass Tausende von pflanzlichen Spezies, die noch vor 20 Jahren teils als toxisch eingestuft wurden - zum Beispiel Selen - wichtige Aufgaben in der Bewahrung unserer Gesundheit innehaben. Insbesondere werden die Carotinoide, Phytosterole, Flavonoide nunmehr als "semiessentiell" eingestuft, und ihnen werden wichtige Gesundheitsfunktionen beigemessen.

Aber auch bei den Spuren- und Ultraspurenelementen, die offiziell noch nicht als essentiell bzw. als semiessentiell definiert sind, ist es gut vorstellbar, dass viele hiervon wichtige enzymatische Funktionen in unserem Stoffwechsel ausüben. Beispielsweise die Enträtselung der Funktion der katalytisch wie enzymatisch hochaktiven Platin-, Gold-, Silber-, Palladiumspezies, die in unseren Organen im dreistelligen ppt-Bereich vorhanden sind, was enzymatisch gesehen hohe Konzentrationen darstellt, sind immer noch nicht aufgeklärt. Unter dem groben analytischen Raster verbergen sich gerade im enzymatischen Bereich wichtige Details, die unser Wissen zum Thema "Gesundheitsnährstoffe" erheblich erhellen könnten.

Wie wichtig spezifische pflanzliche Wirkstoffe für unsere Gesundheit effektiv sind, wird zudem durch eine oftmals geringe Bioverfügbarkeit der jeweiligen Spezies verschleiert; zudem sind Bioverfügbarkeiten auch spezifisch von den jeweils angewendeten Zubereitungsmethoden abhängig, was eine phänomenologisch wie statistische Auswertung sehr schwer macht.

Das Thema Bioverfügbarkeit - also der Anteil eines verzehrten Wirkstoffs, der letztendlich in den Zellen landet, wo er gebraucht wird - ist ein weitgehend noch unergründeter Forschungsbereich. Gerade bei schwerlöslichen Wirkstoffen, und zwar organischer wie mineralischer Natur, ist auf Grund deren schlechten Bioverfügbarkeit bei einseitiger Ernährungsweise bei vielen Menschen ein erheblicher Mangel zu erwarten, der nur schwer wieder zu korrigieren ist.

Andererseits gibt es viele Pflanzen mit einem sehr hohen Anteil an Vitalstoffen, die allerdings hinsichtlich ihrer Zubereitungs-, Verzehr- und Genusseigenschaften zu unattraktiv sind, um weitläufig als Vitalstoffquelle genutzt zu werden. So gehen beispielsweise viele Ölpresskuchen z. B. aus Rapssamen, Sonnenblumenkernen und Leinsamen in die Tierfütterung, obwohl der Vitalstoffgehalt diese Rohstoffe für den Menschen als hochgradig gesund qualifiziert.

Es sei auch erwähnt, dass einige Vitalstoffe, wie dies z. B. beim Lycopin, dem roten Farbstoff der Tomate, der Fall ist, nur nach längerem Kochen bioverfügbar werden und erst dann seine jeweiligen positiven Eigenschaften als z. B. Antioxidans im Körper entfalten kann. Ähnlich steht es mit dem Beta-Carotin der Karotte. Andere Vitalstoffe wie das Vitamin C sind dagegen stark temperaturempfindlich und werden genau unter den Bedingungen zerstört, unter denen andere erst bioverfügbar und wirksam werden.

Es wäre deshalb ernährungsphysiologisch äußerst wünschenswert, wenn man eine Technologie zur Verfügung hätte, die Vitalstoffe möglichst ganzheitlich und in einer wirksamen Form pflanzlichen Rohstoffen entlocken und diese in Form eines leicht verzehrbaren lagerfähigen und schmackhaften Produkts aufkonzentrieren könnte.

Das Schlüsselthema ist hierbei die Bioverfügbarkeit der Vitalstoffe. Diese soll beim Verzehr der eigentlichen Pflanze nach Möglichkeit sogar erhöht werden.

Obwohl es von sehr großem Nutzen für die Qualität unserer Ernährung wäre, gibt es dennoch immer noch kein lebensmitteltechnisches Verfahren, um
a) Vitalstoffe möglichst ganzheitlich und unversehrt zu extrahieren,
b) dabei insbesondere die schwerlöslichen Vitalstoffe in einer hoch bioverfügbaren Form darzubieten und
c) dabei auf den Zusatz von ernährungsphysiologisch fragwürdigen Zusatzstoffen zu verzichten.

Nach dem Stand der Technik werden hauptsächlich alkoholische Extraktionsmethoden herangezogen um die Vitalstoffe aus Pflanzen herauszulösen, wobei diese letztendlich auch in der alkoholischen Phase belassen werden - mit den bekannten Nachteilen des Alkohols als Trägermedium.

Zur Steigerung der Bioverfügbarkeit ist neuerdings der Weg der Überführung organisch-pflanzlicher Wirkstoffe in Nanopartikel bekannt geworden (s. D. Horn u. J. Rieger, "Organische Nanopartikel in wässriger Phase", Angew. Chem. 5, 2001, 113, 4460 - 4492). Ein Lösemittelextrakt, der z. B. Carotinoide enthält, wird schockerhitzt, um die Partikelgröße im Zuge der damit verbundenen Löslichkeitserhöhung zu minimieren. Parallel dazu werden bevorzugt Tenside eingesetzt, um die entsprechenden nanoskalischen Partikel micellenartig zu stabilisieren. Dann folgt die Eindüsung in ein wässriges Medium unter nanoskalischem Ausfällen der Vitalstoffspezies. Schließlich wird die nanoskalische wässrig-organische Suspension mit einer Sprühtrocknung in ein lagerfähiges Granulat überführt; für viele temperaturempfindliche Spezies ist sogar eine Sprüh-Gefriertrocknung notwendig.

Nach DE 102 14 031 A1 werden zur Darstellung der Nanopartikel zuerst homogene Lösungen mit geeigneten Lösemitteln hergestellt, die dann in ein Nicht-Lösemittel - in der Regel Wasser - eingerührt werden, dem wiederum ein Stabilisator beigemengt wurde. Die somit hergestellten Suspensionen werden bevorzugt durch Sprühtrocknung hergestellt und enthalten über 50 % einer spezifizierten Wirksubstanz.

In DE 101 04 494 A1 werden nanoskalische Carotinoide durch Dispergierung mit Lactose und einem Schutzkolloid in wässriger Lösung dargestellt und anschließend sprühgetrocknet. Zur Dispergierung werden die wässrigen Lösungen in der Regel unter Druck bei Temperaturen bis zu 200°C schockerhitzt.

In DE 198 56 432 A1 ist die Herstellung von nanopartikulären Kern-Schale-Systemen beschrieben, wobei die Schale aus einer stabilisierenden Hüllmatrix besteht.

In DE 199 19 751 A1 wird die Herstellung von stabilen wässrigen Dispersionen sowie von stabilen wasserdispergierbaren Trockenpulvern von Xanthophyllen beschrieben. Hierbei werden wiederum die entsprechenden Xanthophylle in einer organischen Phase gelöst, bevorzugt wiederum unter Schockerhitzung und in eine wässrige Phase eingedüst, wobei das Xanthophyll als hydrophobe Phase nanodispers erhalten wird.

In WO 91/06292 wird die Überführung von Carotinoiden in Mikropartikel < 10 µm beschrieben. Hierbei werden entsprechende Carotinoide mit Gelatine im wässrigen Milieu vermahlen und die dabei dargestellte Suspension sprühgetrocknet. Nachteilig ist hier die sehr große Partikelgröße. Erst ab Partikelgrößen < 0,1 µm lässt sich die Bioverfügbarkeit entsprechender Wirkstoffe, die in Näherung proportional der äußeren Oberfläche eines Partikels ist, drastisch erhöhen.

Diese Verfahren haben durchweg den Nachteil, dass Stabilisatoren eingesetzt werden müssen, die die Oberflächenenergie der entsprechenden mikro- oder nanoskalischen Partikel herabsetzen und damit eben auch die Bioverfügbarkeit in der Regel vermindern. Die verwendeten Stabilisatoren sind auch aus ernährungsphysiologischer Sicht eher kritisch zu bewerten.

Nachteilig ist ferner, dass man jeweils von isolierten Vitalstoffen ausgeht, also bereits eine Reinigung vorangestellt hat, die viele wichtige Vitalstoffe, sofern man von pflanzlichen Rohstoffen ausgeht, ausgeblendet hat. Aber gerade die Wechselwirkungen der einzelnen Vitalstoffe untereinander führen vielfach zu einer Agglomerisierung und einer damit beeinträchtigten Bioverfügbarkeit, weshalb die dargelegten Verfahren nicht für die Herstellung von Breitband-Vitalstoffpräparaten geeignet sind. Ein Verfahren, mit dem sich möglichst umfassend eine Vielzahl an schwerlöslichen Vitalstoffen gleichzeitig nanoskalisch darstellen lässt, um insbesondere ganzheitliche, auf Pflanzenbasis beruhende Vitalstoffprodukte herzustellen, ist dem Stand der Technik entsprechend nicht verfügbar.

Ziel der vorliegenden Erfindung war es deshalb, ein Verfahren zu entwickeln, bei dem einerseits ein breites Spektrum an wasserunlöslichen beziehungsweise in verdünnten Lebensmittelsäuren schwerlösliche Vitalstoffe nanoskalisch suspendiert werden, ohne dabei ernährungsphysiologisch unwillkommene Hilfssubstanzen einzusetzen und dabei schlussendlich eine Optimierung der einzusetzenden Energien zu erreichen. Gleichzeitig war die Lagerfähigkeit entsprechender Produkte beziehungsweise Zwischenprodukte zu verbessern.

Diese Aufgabe wird erfindungsgemäß mit den Merkmalen des Patentanspruches 1 und durch die Vorrichtung gemäß Anspruch 11 gelöst.

Die im Folgenden verwendeten Begriffe "Sorptionsmittel" und "Feststoffmatrix" werden synonym verwendet.

Ein Sorptionsmittel ist ein Feststoff der eine nutritive Wirkkomponente entweder absorbiert oder innerhalb einer Pseudophase, also einer Phase, die sich äußerlich/mikroskopisch nicht differenzieren lässt, absorbiert beziehungsweise einlagert.

Der Begriff "sorptives System" wird für ein System, bestehend aus einem Sorptionsmittel und einem Sorbens, also die eingelagerte/absorbierte nutritive Wirkstoffkomponente, benutzt.

Wichtig im Rahmen der Erfindung ist, dass die sorbierten beziehungsweise immobilisierten nanoskalischen beziehungsweise hochenergetischen Spezies reaktiv freigesetzt, mobilisiert, beziehungsweise desorbiert werden. Aus Gründen der besseren Übersichtlichkeit seien diese Freisetzungsmodi im Folgenden integrativ als Desorption oder besser als "Reaktivdesorption" bezeichnet.

Der Ausdruck "nanoskalisch" wird im Folgenden als kleiner gleich 100 nm verstanden; dies ist zugleich Trennscheide unterhalb derer aus ernährungsphysiologischer Sicht quasi unlösliche bzw. schwerlösliche Vitalstoffspezies eine sehr hohe Bioverfügbarkeit zugeschrieben wird.

Die Erfindung stellt somit ein Verfahren zum Herstellen einer trinkfertigen Lösung von Wirk- beziehungsweise Vitalstoffspezies zur Verfügung, das im Einzelnen zunächst das Herstellen einer Basissuspension basischmineralischer Natur umfasst. Nach deren Aufmahlen zusammen mit der Wirkstoffspezies wird dieselbe mit einer sauren Reaktivkomponente kontaktiert und die sorptive Feststoffmatrix löst sich im Rahmen einer Säure-Base-Reaktion auf.

Die Erfindung schafft damit ein Verfahren zur Solubilisierung von Wirkstoffen, insbesondere schwerlöslicher Vitalstoffspezies sowie zur Herstellung ganzheitlich bioverfügbarer Vitalstoffkonzentrate und -lösungen auf Basis lagerfähiger Einzelkomponenten.

Der Anteil der einzelnen Feststoffspezies bezogen auf die Trockenmasse beträgt zwischen 0,01 % und 30 %. Für katalytisch aktive Spuren- und Ultraspurenelemente wie Gold, Silber, Platin, Wolfram betragen entsprechende Beladungen teilweise jedoch nur zwischen 10 und 1.000 ppb - wobei diese Werte allerdings im katalytisch-enzymatischen Bereich als relativ hohe Beladungen anzusehen sind.

Die in einem genießbaren, das heißt lebensmitteltypischen pH-Bereich zwischen 3 und 7 in wässriger Lösung schwerlöslichen bzw. unlöslichen Wirk-/ Vitalstoffspezies, deren Bioverfügbarkeit gesteigert werden soll, wird im ersten Schritt in einer Basislösung bzw. Basissuspension vorgelegt und im 2. Schritt nanoskalisch bzw. molekulardispers an/in einer sorptiven Matrix gebunden.

Die sorptiven Matrices sind im Wesentlichen Basen, so dass diese im abschließenden Prozessschritt anhand einer Säure-Base-Reaktion mit einer entsprechenden sauren Reaktivkomponente spontan aufgelöst werden kann, unter ebenso spontaner Freisetzung der sorbierten Wirk-/Vitalstoffspezies. Wesentlich im Gesamtprozess ist, dass die Desorption/Freisetzung der sorbierten Wirk-/Vitalstoffspezies sehr rasch bzw. spontan erfolgt, soll dabei die nanoskalische Wirkstoff-/Vitalstofffstruktur nicht verloren gehen. Denn Reagglomerierungsprozesse sind in der Regel kinetisch - d. h. sind zeitgesteuert und verlaufen an Sorbentien vielfach anhand von Oberflächendiffusionseffekten ab.

In einer bevorzugten Ausführungsform der vorliegenden Erfindung wird deshalb die sorptiv wirkende Oberfläche des Sorbens sehr schnell abgetragen, ohne dass es auf Grund lokaler Aufkonzentrierung an der Oberfläche des Sorbens zur Reagglomerisation/Rekristallisation der sorbierten Spezies kommen kann.

Die spontane Auflösung der sorptiven Oberfläche hat des Weiteren den Vorteil, dass Oberflächenladungen in möglichst großem Umfang auf die sorbierten Spezies übertragen werden, wodurch die dann desorbierten Spezies, die ansonsten a priori instabil sind und zur Agglomeration neigen, sich tendenziell wegen Ladungsgleichheit abstoßen und ihre innewohnende Agglomerisierungstendenz deutlich zurückgedrängt wird.

Typischerweise verläuft deshalb das Auflösen der sorptiven Matrix im Konzentrat schneller als in der verdünnten Lösung. Die Reduzierung der Flüssigkeitsmenge im Auslöseprozess ermöglicht es, die mechanische Mischenergie besser in die sorptive Matrix, welche viskos und insbesondere thixotrop ist, einzubringen. Auch aus Gründen der effizienteren Diffusion des sich durchdringenden Säure-Base-Systems ist ein hochkonzentriertes System vorteilhaft.

Typischerweise sind in/an der sorptiven Matrix nanoskalische Partikel sorbiert, die im Herstellungsprozess des sorptiven Systems generiert werden. Im Folgenden wird deshalb auch von sorbierten "rudimentären Nanopartikeln" gesprochen.

Im Unterschied zum Stand der Technik wird primär kein Fokus auf eine stabilisierende Formulierung gelegt, die ja immer auch zu Lasten der Bioverfügbarkeit geht, sondern der Fokus liegt hier darauf, die Freisetzung der Nanopartikel zeitlich flexibel, dann aber möglichst rasch erfolgen zu lassen. Typisch ist, dass die dargestellten kolloidal gelösten Nanopartikel in der entsprechenden Lösung a priori instabil sind, d. h., sie neigen zum Agglomerieren und büßen dabei einen wesentlichen Teil ihrer Bioverfügbarkeit, die in Näherung proportional der äußeren Oberfläche der Primärpartikel ist, ein.

Mit Hilfe des Verfahrens der nanoskalischen Reaktivdesorption lassen sich ausgewogene Wirkstoff- bzw. Vitalstoffkonzentrate mit sehr hoher biologischer Wertigkeit nanoskalisch darstellen. Kern ist eine Kombination einer Ad-/ Absorptionsstufe mit einer gesteuerten Desorptionsstufe. In der Ad-/ Absorptionsstufe wird die Wirkstoff-/Vitalstoffspezies bereits rudimentär nanoskalisch erzeugt und vor Agglomerisierung geschützt. In der Desorptionsstufe wird das Sorbens infolge einer Säure-Base-Reaktion zumindest partiell aufgelöst. Parallel dazu wird die sorbierte Spezies nanoskalisch bzw. molekulardispers freigesetzt.

Durch die Überlagerung der Desorption mit den elektronischen Effekten der Reaktion können Spezies, die a priori stark zur Agglomerisierung neigen, nanoskalisch dispergiert werden. Diese Freisetzung bzw. Aktivierung der nanoskalischen Wirk-/Vitalstoffe erfolgt vorzugsweise unmittelbar vor dem Konsum bzw. der Applizierung des Anwenders, insbesondere der peroralen Aufnahme. Das Verfahren ist vorteilhaft im Rahmen eines Extraktionsprozesses sowie der Saftgewinnung einzubinden. Mit dem erfindungsgemäßen Verfahren lassen sich Vitalstoffe pflanzlichen und/oder tierischen Ursprungs aufkonzentrieren und in hochgradig bioverfügbarer Form darreichen.

Prinzipiell lassen sich anhand des nanoskalischen Reaktivdesorptionsverfahrens, im Folgenden NRD-Verfahren genannt, die Bioverfügbarkeit jedes tendenziell schwerlöslichen Wirkstoffs/Vitalstoffs erhöhen. Das erfindungsgemäße Verfahren fokussiert insbesondere auf jene im pH-Bereich zwischen 3 und 7 bzw. in verdünnter Säure schwerlöslichen pflanzlichen Wirk- bzw. Vitalstoffspezies. Diese lassen sich in folgende Klassen unterteilen:
A. Spurenelemente, wie Eisen, Kobalt, Kupfer, Molybdän, Silizium, Aluminium, Mangan, Chrom, Bor, Nickel, Zink, Vanadium, Selen.
B. Ultraspurenelemente, wie Titan, Zirkon, Rubidium, Lithium, Yttrium, Cer, Palladium, Lanthan, Neodym, Silber, Wolfram, Gallium, Tellur, Thorium, Praseodym, Niob, Samarium, Gadolinium, Dysprosium, Arsen, Scandium, Indium, Antimon, Cäsium, Germanium, Ytterbium, Erbium, Europium, Bismut, Platin, Tantal, Terbium, Holmium, Rubidium, Beryllium, Gold, Iridium, Rhenium, Ruthenium, Palladium und Rhodium.
C. Sekundäre Pflanzenstoffe, wie beispielsweise die
   a. Phytosterine: β -Sitosterin
   b. Flavonoide, wie Spiraeosid, Isoquercitrin, Flavonolignane, Rutosid, Hyperosid, Catechingerbstoffe, Procyanidin B-2, Hesperidin, Genistin, Naringin, Vitexin, Orientin, Genistin, Ononin
   c. Carotinoide, wie alpha-Carotin, β-Carotin, Lycopin, Zeaxanthin, Astaxanthin, Canthaxanthin, Cryptoxanthin, Lutein, Neoxanthin, Phytofluen, Phytoin, Rubixanthin, Violanxanthin, Fucoxanthinol, Violaxanthin, Flavoxanthin
   d. Protease-Inhibitoren
   e. Glucosinolate, wie Isothiozynate, Thiozyanate und Indole
   f. Saponine
   g. Phytoöstrogene, insbesondere den
      i. Isoflavonen, wie Genistein, Daidzein, Glycetein, Biochanin A, Formononetin
      ii. Lignanen, wie Secoisolariciresinol und Matairesinol
      iii. Coumestanen, wie Coumestrol und A'-Methoxycoumeastrol
D. Vitamine, wie Retinol, Calciferol, Tocopherol, Phyllochinon

Das NRD-Verfahren hat deshalb einen erheblichen Nutzen in der Formulierung pharmakologischer Präparate, insbesondere auf Basis a priori schwerlöslicher Wirkstoffe.

Es sei ferner bemerkt, dass die Gegenwart, insbesondere die Coadsorption/ die Coabsorption von gut löslichen Spezies an/in der sorptiven Matrix, den Gesamtprozess nicht stören, und dass diese im Rahmen einer ganzheitlichen Darstellung eines Vitalstoffproduktes sogar erwünscht ist.

Das Verfahren eignet sich besonders auch zur Steigerung der Bioverfügbarkeit von Multikomponenten-Wirkstoff- und -Vitalstoffgemischen, da hier die hohe sorptive Wirkung des stabilisierenden Sorbens die attraktiven Wechselwirkungen zwischen den Wirkstoffspezies überlagert, und damit Segregation in den Hintergrund drängt.

Ein wichtiges Merkmal ist die Bereitstellung von vitalstoffreichen Reaktivkomponenten in Form eines sorptiven Systems, in denen die einzelnen, darin enthaltenen Vitalstoffe lagerstabil sind, und zwar mindestens 10 Tage, bevorzugt mindestens 100 Tage, typischerweise aber mehr als 500 Tage, wobei eine Agglomerisierung der einzelnen Vitalstoffe nur in geringem Umfang eintritt.

In dieser Lagerphase nimmt der mittlere Durchmesser, der dann freigesetzten nanoskalischen Vitalstoffpartikel typischerweise um nicht mehr als 100 % zu, bevorzugt um weniger als 50 %. Das bedeutet, innerhalb der Speichermatrix findet ein äußerst geringes Wachstum beziehungsweise eine äußerst geringe Agglomeration der Partikel statt.

Besonderes Merkmal ist es, die Vitalstoffe, die besonders zur Agglomerisation neigen, in einer bioverfügbaren Form "einzufrieren" und damit auch außerhalb einer Pflanzenzelle zu stabilisieren. Es darf angenommen werden, dass in einer Pflanzenzelle voraussichtlich Ähnliches passiert. Schwerlösliche beziehungsweise zur Agglomerisation neigende Vitalstoffspezies werden durch die Zellmembran der Pflanzenzellen voneinander getrennt und können damit nicht agglomerieren beziehungsweise kristallisieren. Erst in dem Moment, an dem durch die mechanische Zerstörung der Zellmembran, das "Agglomerisations-Hindernis" nicht mehr besteht, können "Kristallkeime" beziehungsweise "nanoskalische Partikel" gegenseitig "andocken" und agglomerieren.

In Ausführungsformen hergestellte instabile Nanopartikel zeigen ein Anwachsen des Partikeldurchmessers in der Regel von mindestens 5 %, typischerweise mindestens 50 % pro Stunde, bezogen auf den anfänglichen Durchmesser, direkt nach der Reaktivdesorption; absolut wächst der Partikeldurchmesser zwischen 5 nm - 5 µm pro Stunde in der trinkfertigen Lösung an. Das bedeutet, in der "aktivierten" Dispersion findet ein starkes Wachstum beziehungsweise eine starke Agglomeration der Partikel statt. Dieses Wachstum wird durch ein Kontaktieren in Ruhe positiv beeinflusst.

Eine trinkfertige Lösung umfasst die mit dem Verfahren hergestellten Nanopartikel, wobei der Konzentrationsbereich in dem Bereich, welcher typischerweise isotonisch ist, insbesondere bei etwa 2.000 bis 10.000 mg/l Mineralien liegt.

Die Bestimmung der Partikelgrößenverteilung, insbesondere des Durchmessers, erfolgt über Laser-Streuversuche oder anhand der direkten Vermessung der Partikel, die aus stark verdünnten Lösungen auf geeigneten Membranen abgeschieden werden, mit Hilfe eines Elektronentransmissionsmikroskops.

Bevorzugt wendet man die Trinklösung innerhalb einer Stunde nach entsprechender Herstellung/Aktivierung an.

Die sorptiven Komponenten sind dabei mineralischer Natur. Als mineralisches Sorbens fungieren bevorzugt Kalziumhydroxid, Kalziumcarbonat, Kalziumhydrogencarbonat, Kalziumoxid, Magnesiumoxid, Magnesiumhydroxid, Magnesiumcarbonat, Magnesiumhydrogencarbonat sowie entsprechende Kaliumsalze bzw. Mischungen daraus.

Als organisches Sorbens werden bevorzugt Fruchtsäuren wie Zitronensäure, Apfelsäure, Weinsäure, Bernsteinsäure, Ascorbinsäure, aber auch Zucker wie Fructose, Glucose, Maltose, Dextrose bzw. deren Mischungen verwendet. Zudem lassen sich Milchsäuren, Asparagin- und Glutaminsäuren verwenden. Besonders günstig ist, wenn das sorptive System innig gemahlen wird. Das Sorbens wirkt hierbei als hoch effektiver sekundärer Mahlkörper, der das Sorptiv an
seinen Kristalloberflächen in extrem dünnen Schichten anlagert und im Verlauf des Mahlprozesses dieses dann im Rahmen seiner eigenen Zergliederung auch in einer mehr oder minder ausgeprägten amorphen Struktur einlagert. Hierzu können in einem Trockenprozess, der bevorzugt bei einem organisch-sorptiven System verwendet wird, übliche Kugelmühlen eingesetzt werden. Ein mineralisch-sorptives System, das höhere Gitterenergien aufzubieten hat, wird dagegen bevorzugt wässrig, bevorzugt mit einer Perlmühle, aufgemahlen.

Benutzt man auch bei einem organisch-sorptiven System *ein Mahlen* in flüssiger Phase, so ist darauf zu achten, dass das Sorbat eine möglichst geringe Löslichkeit in der Flüssigkeit besitzt, um dessen partikuläre Struktur nicht zu zerstören. Hierbei wird durch Herabsetzen der Oberflächenenergie in Folge der Benetzung der Mahlprozess in seiner Effizienz erheblich gesteigert. Organische sorptive Systeme werden deshalb in einer vorteilhaften Ausprägung des erfindungsgemäßen Verfahrens in einem wässrigen und/oder alkoholischen Medium gemahlen.

Die Reaktivdesorption wird bevorzugt anhand einer Säure-Base-Reaktion gesteuert, da diese sehr schnell abläuft und dabei die Nanopartikel ungehindert freigesetzt werden. Der pH-Wert der basischen Komponente beziehungsweise deren wässriger Lösung ist bevorzugt größer 10, da hierbei beispielsweise sorbierte Spurenelemente wie Eisen-, Kobalt-, Kupfer-, Molybdänspezies sehr gut speicherbar sind und wenig zur Agglomerisation neigen. Gleiches gilt für Silizium, Aluminium, Mangan, Chrom, Bor, Titan, Nickel, Zink, Vanadium, Selen, Zirkon, Rubidium, Yttrium, Cer, Palladium, Lanthan, Neodym, Silber, Wolfram, Gallium, Tellur, Thorium, Praseodym, Niob, Samarium, Gadolinium, Dysprosium, Arsen, Scandium, Indium, Antimon, Cäsium, Germanium, Ytterbium, Erbium, Europium, Bismut, Platin, Tantal, Terbium, Holmium, Rubidium, Beryllium, Gold und Rhodium.

Umgekehrt ist die saure Komponente bzw. deren wässrige Lösung im pH-Bereich bevorzugt kleiner 5 angesiedelt. Der saure pH-Wert wirkt antibakteriell und erhöht somit die Lagerfähigkeit des Systems. Zudem erhöht wiederum eine möglichst große pH-Differenz der beiden Komponenten eine möglichst spontane Reaktivdesorption mit den beschriebenen Vorteilen.

Besonders vorteilhaft ist auch die Verwendung von Zuckerstoffen und Dispergier- beziehungsweise Trockenhilfsmitteln wie Pektine, Gelatine, Maltose neben der Fruchtsäure.

Als Rohstoffe werden bevorzugt Obst und Gemüse, insbesondere solche mit hohem Vitalstoffgehalt, wie Sonnenblumenkerne, Raps, Gerstengras, Feigenkakteen, Inka-Gurken, Rotklee, Sojabohnen, Kudzu, Gerste, Weizen, Kohl, Leinsamen, Grüner Tee, Kirschen, Heidelbeeren, Preiselbeeren, Erdbeeren, Olivenblätter, Rotkohl, Zwiebeln, Weintrauben, Rotwein, Spargel, Yucca, Sanddornbeeren, Blasentang, Goldrutenkraut, Arnikablüten, Blattpetersilie, Eisenkraut, Löwenzahnblätter, Holunderbeeren, Eleutherococcuswurzel, Ringelblumenkraut, Ginseng, Rosenblätter, Blütenpollen, Malvenblüten, Grünkohl, Mariendistel, Weinrautenkraut, Weißdorn, Stiefmütterchen, Odermenning, Hauhechel, Passionsblume, Weizenkleie, Reisschalen, Lupinen, Oliven, Distel und Sesam eingesetzt. Vorteilhaft werden insbesondere auch Presskuchen entsprechender Samen und Früchte eingesetzt. Weiterhin ist die Verwendung von Karotten, Äpfeln, Kartoffelschalen, Trauben, optional auch als Trester, sowie Nussschalen beziehungsweise Kleie der Haselnuss, Walnuss, Macadamianuss, Kokosnuss, Mandel, Kürbiskerne, Traubenkerne und dergleichen, Ölsamen, vor allem auch Weizen- und Reiskleie sowie die Rückstände aus Zuckerrohr und Rübenzucker, die in der Zuckergewinnung anfallen, geeignet. Typischerweise werden als Rohstoff auch Fruchtschalen, insbesondere Orangen-, Zitronen-, Kartoffel- und Zwiebelschalen verwendet.

In einer besonders bevorzugten Ausführungsform der Erfindung wird der Rückstand der wässrigen beziehungsweise organischen Extraktion vollständig oxidiert und das Oxidat in eine basische Suspension überführt. Optional lässt sich zur weiteren Verarbeitung auch Holzasche und/oder Kohleasche verwenden. In einer besonders bevorzugten Ausführungsform der Erfindung wird der Rückstand einer wässrigen beziehungsweise organischen Extraktion des Rohstoffs beziehungsweise der Rohstoffe vollständig oxidiert und das Oxidat in eine basische Suspension überführt. Diese basische Suspension lässt sich idealerweise als basische Reaktionskomponente in der Reaktivdesorption der organischen Nanopartikel des organischen Sorbens verwenden.

Neben der Freisetzung von nanoskalischen organischen Vitalstoffen werden bei letztgenannter Vorgehensweise gleichzeitig auch mineralische Nanopartikel, die in der basischen Suspension im Wesentlichen an mineralischen, insbesondere erdalkalischen Spezies sorbiert sind, freigesetzt. Die basische Suspension stellt somit als Reaktivkomponente wiederum ein sorptives System dar, das nanopartikuläre Spuren- und Ultraspurenelemente freisetzt.

Somit werden einerseits die in dem entsprechenden Rohstoff eingelagerten schwerlöslichen Mineralstoffe, beispielsweise Eisen, Silber, Gold, Platin, Bismut, Kobalt, Kupfer, nanoskalisch freigesetzt, typischerweise in Form von multielementaren Nanopartikeln. Andererseits werden auch die an dem organischen Sorbens sorbierten organischen Nanopartikel freigesetzt, wodurch ganzheitliche, bioverfügbare Vitalstofflösungen dargestellt werden.

Im Rahmen der stattfindenden Säure-Base-Reaktion der im Wesentlichen alkalischen und erdalkalischen Carbonate, Hydrogencarbonate, Hydroxide, Oxide mit der Säurekomponente werden letztere in die entsprechenden anionischen Säurereste - zum Beispiel Citrate, Lactate, Asparagate, und so weiter - überführt, die im Körper zu Carbonat verstoffwechselt werden und damit als wichtige Spezies agieren, um der von vielen Medizinern als gefährlich und krankmachend eingestuften Azidose entgegenzuwirken und dabei ein ausgewogenes Säure-Base-Gleichgewicht etablieren.

Der Extraktionsprozess wird erfindungsgemäß derart durchgeführt, dass vor allem jene Spezies, denen ein hoher Gesundheitsnutzen zugeschrieben wird wie den Carotinoiden, Flavonoiden, Phytoöstrogenen, Alkaloiden etc., möglichst weitgehend extrahiert werden. Als organische Lösemittel lassen sich Ketone, Alkohole, Ester, Ether, Amine benutzen, insbesondere Aceton, Ethanol, Methanol, Methylethylketon, N-Methylpyrolidon, Isopropanol und Tetrahydrofuran verwenden. Ferner kann die Extraktion wässrig erfolgen.

Vorteilhaft ist auch die Extraktion unter Verwendung von Fruchtsäuren, womit besonders auch die Alkaloide und Mineralien/Spurenelemente angereichert werden. Bei einer wässrigen Extraktion macht man sich typischerweise Temperatureffekte zu Nutze, um die Löslichkeit der Vitalstoffspezies zu erhöhen und damit die Extraktion zu beschleunigen. Anhand eines Fruchtsäurezusatzes zum Extraktionsmittel lassen sich zudem der mineralische und der Alkaloid-Anteil spezifisch erhöhen.

Der Trockenprozess kann dabei über eine Sprühtrockenstufe erfolgen. Bevorzugt wird auch ein beheiztes Drehrohr benutzt, wobei die sich an den Wandungen festsetzenden Beläge über einen Schabmechanismus temporär entfernt werden, die dann vorzugsweise in einem Trockenmahlprozess zu einem feinen Pulver weiterverarbeitet werden.

Die Sprühtrocknung hat den Vorteil hinsichtlich einer unerwünschten Agglomerisation, da hierdurch der Übergangszustand zwischen gelöstem und sorbiertem Zustand zeitlich verkürzt und damit auf ein Minimum reduziert werden kann.

Um insbesondere die oxidationsempfindlichen SPS und Vitamine während der thermischen Belastung im Prozess nicht zu oxidieren, werden bevorzugt als Oxidationsinhibitor Vitamin C, Vitamin E bzw. Tocopherol, dem Extrakt bzw. dem zu extrahierenden Rohstoff beigemengt.

In einem Beispiel werden bei dem Verfahren drei Fraktionen von mindestens einer Ursprungspflanze, die zwischenzeitlich separiert voneinander gewonnen werden, wieder zusammengeführt.

In einer vorteilhaften Weiterbildung wird bei der Durchführung von Schritt c die nanoskalische Wirk-/Vitalstoffdispersion auf sorbierende und/oder immobilisierende Lebensmittel aufgebracht und/oder in diese eingebracht. Beispielsweise ist im Rahmen der Erfindung vorgesehen, dass die nanoskalische Wirk-/ Vitalstoffdispersion auf Getreideprodukte, insbesondere Mehl, Cerealien, Hartweizengrieß, Kakaopulver, Zucker, Milchpulver und Stärke aufgebracht und/oder in diese eingebracht wird. Die nanoskalische Wirk-/Vitalstoffdispersion kann jedoch auch in gelierfähige und/oder gelatinehaltige und/oder pektinhaltige Lebensmittel, wie beispielsweise Marmelade, Konfitüre und/oder Fruchtgummis aufgebracht und/oder in diese eingebracht werden.

Um für den Anwender eine besonders einfache Durchführung des Verfahrens zu ermöglichen, werden in einer weiteren vorteilhaften Ausführungsform des Verfahrens, im Stadium der Konfektionierung, die Reaktivkomponenten mit Hilfe eines Mehrkammerbehältnisses, insbesondere mit Hilfe einer Mehrkammertube, zusammengebracht. Die Kammern dienen dabei als Reservoir für die Komponenten.

Zur Herstellung von Wirk-/Vitalstoff-angereicherten Lebensmitteln, insbesondere von sogenanntem "functional food", sieht eine Ausführungsform vor, dass in einem Verfahren wie oben beschrieben, ein Wirk-/Vitalstoffkonzentrat hergestellt wird,
und dass dieses Wirk-/Vitalstoffkonzentrat nach seiner Herstellung zeitnah, insbesondere nach weniger als 5 Stunden, bevorzugt nach weniger als 1 Stunde, besonders bevorzugt weniger als 5 Minuten nach der Durchführung von der Konfektionierung auf ein sorbierendes und/oder immobilisierendes Lebensmittel aufgebracht und/oder in ein sorbierendes und/oder immobilisierendes Lebensmittel eingebracht wird.

Die Durchführung des Verfahrens gelingt dann besonders zuverlässig, wenn eine strukturviskose, insbesondere eine thixotrope Speichermatrix eingesetzt wird. Um diese zur Verfügung zu stellen, schafft die Erfindung ein Verfahren zur Herstellung einer strukturviskosen, insbesondere einer thixotropen und säurelöslichen Speichermatrix, insbesondere zur Verwendung als sorptive und/oder immobilisierende Feststoffmatrix des oben erläuterten Verfahrens, wobei ein Ca- und/oder Mg-haltiges Mineral in Anwesenheit von Wasser gemahlen wird und vor oder während des Mahlprozesses ein flüchtiges und/oder reaktives Dispergiermittel zugesetzt wird.

Dieses Beispiel schafft mit dem Verfahren zur Herstellung einer strukturviskosen, insbesondere einer thixotropen und säurelöslichen Speichermatrix zudem eine thixotrope und säurelösliche Speichermatrix, welche hauptsächlich Wasser und Ca und/oder Mg in hydroxidischer und/oder Carbonat-gebundener Form enthält. Insbesondere liegt der Wassergehalt in einer vorteilhaften Ausführungsform im Bereich zwischen etwa 95 % und etwa 50 %, bevorzugt zwischen etwa 90 % und etwa 75 %, bezogen auf die Trockenmasse.

In einer vorteilhaften Weiterbildung hat die Speichermatrix bei 30°C eine Thixotropie größer 100.000 mPa·s, bevorzugt größer 300.000 mPa·s und besonders bevorzugt größer 1.000.000 mPa·s.

Die Dichte der Speichermatrix liegt zwischen etwa 1,03 und etwa 1,65, bevorzugt zwischen etwa 1,05 und 1,3 g/cm³. Die Viskosität beträgt bei einer Temperatur von 30°C +/- 5°C zwischen etwa 20 mPa·s und etwa 50.000 mPa·s, bevorzugt zwischen etwa 3.000 mPa·s und etwa 12.000 mPa·s.

Die thixotrope Speichermatrix weist in einem bevorzugten Beispiel neben Wasser und amorphen anorganischen Spezies hauptsächlich die Mineralien Calcit und/oder Portlandit und/oder Brucit und/oder Dolomit auf. Die thixotrope Speichermatrix kann zur nanoskalischen Speicherung von Spuren- und Ultraspurenelementen verwendet werden.

Die Erfindung bietet mit dem Verfahren zur Solubilisierung von Wirkstoffen und dem Verfahren zur Herstellung einer strukturviskosen, insbesondere einer thixotropen und säurelöslichen Speichermatrix, insbesondere zur Verwendung als sorptive und/oder immobilisierende Feststoffmatrix zur Solubilisierung von Wirkstoffen eine Möglichkeit, ein für die Gesundheit des Menschen wertvolles Produkt zu schaffen.

Die einzelnen Ultraspurenelemente liegen dabei erfindungsgemäß in einem Gewichtsverhältnis, bezogen auf Kalzium plus Magnesium, als Standard zwischen 0,1 ppm und 1.000 ppm, bevorzugt zwischen 1 ppm und 100 ppm vor. In der trinkfertigen Lösung liegen insbesondere die zur Klasse der Ultraspurenelemente zählenden katalytischen Metalle wie Silber, Gold, Platin, Palladium, Rhodium, Wolfram, Ruthenium, Rhenium, etc., die in eingebundenen Komplexen bzw. Enzymen als atomare bzw. ionische Einheit wirken, in einem Konzentrationsbereich zwischen 10¹⁴ (1E14) und 10¹⁷ (1E17) Atome pro Liter vor, d. h. auf einer enzymatisch-katalytischen Skala im signifikanten Konzentrationsbereich. Typischerweise, bei Einsatz hochwertiger pflanzlicher Rohstoffe, liegen zwischen 15 bis 35 Ultraspurenelemente in diesem Konzentrationsbereich vor. Großer Vorteil des Verfahrens zur Herstellung einer strukturviskosen, insbesondere einer thixotropen und säurelöslichen Speichermatrix, insbesondere zur Verwendung als sorptive und/oder immobilisierende Feststoffmatrix in einem weiteren Schritt des Verfahrens zur Solubilisierung von Wirkstoffen ist, dass in dem sorbierenden beziehungsweise immobilisierenden System die mechanisch eingebrachte Dispergierenergie gespeichert bleibt. Die rudimentäre Dispersion der Vitalstoffspezies bleibt damit erhalten. Die zum Mischen der beiden Makrokomponenten, das sorbierende beziehungsweise immobilisierende System einerseits sowie die Reaktivkomponente andererseits, benötigt dann besonders geringe, insbesondere eine um Größenordnungen geringere Mischenergie, die üblicherweise durch Schütteln, einfaches Rühren und/oder gemeinsames Durchwalken/Durchkneten erbracht werden kann.

Schlussendlich wird der Anwender des Verfahrens in den Zustand versetzt, als hätte er vor Ort ein Hochleistungs-Dispergierelement - wie eine Perlmühle - mit der er geräte- und ortsunabhängig hochenergetische beziehungsweise nanoskalische Dispersionen herstellen kann.

Ein besonders vorteilhaftes Merkmal des Verfahrens zur Herstellung einer strukturviskosen, insbesondere einer thixotropen und säurelöslichen Speichermatrix, insbesondere zur Verwendung als sorptive und/oder immobilisierende Feststoffmatrix im Verfahren zur Solubilisierung von Wirkstoffen ist die Ausbildung von einer sorbierenden beziehungsweise immobilisierenden Suspension, die Mikro- und/oder Submikrospezies umfasst, die vornehmlich über elektrostatische und/oder dipolare, insbesondere auch über Wasserstoffbrückenbindungen assoziieren.

Unter dem Begriff "immobilisierendes System" ist dabei insbesondere ein System, insbesondere auch ein wässriges System, bestehend aus einer Matrix - im Folgenden auch Speichermatrix genannt - und einer oder mehrerer Einlagerungsspezies zu verstehen, die in der Matrix im Wesentlichen immobilisiert sind. Beispielsweise kann es sich bei der Speichermatrix um lose oder weniger lose Agglomerate handeln, die untereinander ein dreidimensionales Netzwerk bilden, das über Wasserstoffbrückenbindungen oder dipolare Wechselwirkungen zusammengehalten wird. In den Zwischenräumen dieses Agglomerat-Netzwerkes können nun die Einlagerungsspezies eingelagert bzw. zwischengespeichert werden. Diese Zwischenspeicherung erfolgt in einer Art und Weise, in der die Einlagerungsspezies ihre Mobilität weitgehend einbüßen; in jedem Fall aber sind die Zwischenräume des Agglomerat-Netzwerkes hinreichend klein, so dass die Einlagerungsspezies ihrerseits wiederum nicht genügend Platz bzw. Reaktionsraum vorfinden, um agglomerieren zu können.

Aus diesem Grund ist es vorteilhaft, über den bereits beschriebenen Mahlprozess die Primärpartikel der Speichermatrix hinreichend klein darzustellen, damit diese ihrerseits indirekt ein Netzwerk von Hohlräumen, im Folgenden "Cavities" genannt, bilden, in die die einzulagernden Nanopartikel zwar hineinpassen, die aber eben auch genügend klein sind, damit diese nicht genügend Raumvolumen vorfinden, um zu agglomerieren.

In dem NRD-Verfahren kommt der Energetik des Agglomerat-Netzwerks besondere Bedeutung zu. Einerseits müssen die entsprechenden Primärspezies untereinander genügend stark wechselwirken beziehungsweise aneinander physikalisch binden, um eine sogenannte Cavity-Struktur auszubilden, die die Einlagerungsspezies hinreichend stark einengt, damit diese in ihrer Mobilität, in jedem Fall aber in ihrer Fähigkeit stark eingeschränkt sind, sich relativ zueinander derart auszurichten, damit diese selbst Agglomerate ausbilden können. Andererseits muss das Agglomerat-Netzwerk der Speichermatrix genügend instabil sein, damit es in einem sehr kurzen Zeitintervall gelöst werden kann.

Die Speichermatrix besteht beispielsweise aus positiv geladenen Primärpartikeln, die eine Cavity-Struktur ausbilden. In dem Cavity befindet sich hauptsächlich Wasser - mit einem Anionen-Überschuss - beispielsweise Hydroxid- und/oder Carbonat- und/oder Hydrogencarbonat-Ionen.

Die positiv geladenen Primärpartikel können in einer Weiterbildung insbesondere überwiegend aus Ca-/Mg-Hydroxiden bestehen. Auf Grund der hohen Mobilität der Hydroxid-Ionen, welche in wesentlichem Umfang das Agglomerat beziehungsweise die Speichermatrix elektrostatisch und/oder mechanisch stabilisieren, bricht das Agglomerat typischerweise in Bruchteilen einer Sekunde zusammen, wenn dieses mit Säure kontaktiert wird.

Besonders schnell bricht das Agglomerat zusammen, wenn die Säure-Kontaktierung mit dem Einbringen von mechanischer Energie kombiniert wird. Hierbei bricht das Agglomerat durch dessen mechanische Bewegung teilweise - zumindest für einen kurzen Zeitpunkt - zusammen, womit eine erhöhte Mobilität der Hydroxid-Ionen einhergeht. Besonders vorteilhaft benutzt man als Kontaktierungsmittel eine feste Säure - wie Zitronensäure, Apfelsäure, Vitamin C, etc. -, da hiermit eine hohe Dichte eines Hydroxid-Ionenakzeptors beziehungsweise Protonendonators in die Speichermatrix eingebracht wird, die somit den Zusammenbruch des Agglomerats nebst dessen chemischer Auflösung bewirkt.

Im Gegensatz dazu lässt sich die Kontaktierung natürlich auch mit einer wässrigen Säurelösung herbeiführen, allerdings neigt das System hierbei dazu, die Agglomerate nicht vollständig zu lösen bzw. nur langsam zu lösen. Vorteilhaft bei der Kontaktierung durch feste Säurepartikel ist zudem, dass von außen eingebrachte mechanische Energie, die die Durchmischung der Systeme herbeiführen soll, primär in die viskose/thixotrope Matrix mündet und nicht in ein niederviskoses wässriges System. Dieser Vorteil ist nicht zu unterschätzen, da ja mögliche vorteilhafte Anwendungen des erfindungsgemäßen Verfahrens gerade darin liegen, mit einem Minimum an mechanischem Aufwand und unabhängig vom Ort der Anwendung eine nanoskalische Dispersion herzustellen.

Es ist jedoch zu bemerken, dass obwohl natürlich die thixotrope Speichermatrix an sich schon einen oder mehrere Vitalstoffe umfassen kann, die entsprechende Menge nicht in die Zahlen der hier ausgeführten Vitalstoffbeladungen eingeht.

Ein besonderer Vorteil der thioxtropen Speichermatrix besteht darin, dass in einem Mahlprozess die nanoskalisch zu dissipierende Vitalstoffspezies in dem Moment der Herstellung sofort in die Cavity-Struktur eingebunden und damit vor der Reagglomerisierung geschützt wird. Dies ermöglicht einen sehr effizienten Mahlprozess und eine hohe nanoskalische Anreicherung.

Die Herstellung der thixotropen Speichermatrix erfolgt primär in einer innigen Mahlstufe - bevorzugt in einer Perlmühle. Unterstützt wird der Herstellungsprozess durch vorausgehende Sinterung der Kalzium- und Magnesiumsalze. Bevorzugt werden hierbei die entsprechenden Oxide, Hydroxide, Carbonate und Hyrogencarbonate eingesetzt. Das Ca-/Mg-Verhältnis beträgt dabei zwischen 1:50 und 10:1, bevorzugt zwischen 1:5 und 5:1. Als mineralische Spezies wird die thixotrope Speichermatrix typischerweise vorwiegend aus Calzit, Portlandit, Brucit und Dolomit gebildet.

Wesentlich ist, damit die letzte Stufe des Verfahrens zur Solubilisierung von Wirkstoffen, das heißt die Reaktivdesorption, von einem Anwender in einfacher Weise selbst durchgeführt werden kann, dass in einer geeigneten Verpackung die Zusammenführung der Komponenten für die Reaktivdesorption erfolgt.

Vorteilhaft ist die getrennte Aufbewahrung des sorptiven Systems und der Reaktivkomponenten in einer Verpackungseinheit. Vorteilhaft ist insbesondere die örtliche Separierung beider Komponenten durch eine Trennkomponente, wobei durch kurzzeitiges Ausüben einer Kraft beziehungsweise eines Drehmoments, die indirekt auf die Trennkomponente wirkt, diese sich öffnet beziehungsweise bricht. Über die so erzeugte Öffnung ist ein Kontakt möglich und ein Vermischen der beiden Komponenten kann herbeigeführt werden.

Darauf bezugnehmend ist ein Zweikammersystem besonders geeignet, wenn beide Komponenten durch ein Siegel voneinander getrennt sind, das sich auf definierten Druck öffnet und ein gutes Durchmischen beider Komponenten zulässt.

In einer vorteilhaften Ausführung werden sogar drei Extraktfraktionen gewonnen, die dann in einem Dreikomponentensystem abgepackt werden. In diesem Fall würde man bevorzugt
a) ein wässrig-saures Extrakt,
b) eine leicht dispergierbare Trockensubstanz, die im Wesentlichen aus Fruchtsäure als sorptive Feststoffmatrix besteht, an der pflanzliche, organisch extrahierbare Stoffe sorbiert sind, und
c) ein basisches Pulver bzw. eine basische Suspension in einer Verpackungseinheit, durch mindestens zwei Siegel voneinander separiert, zusammenbringen.

Das durch Öffnen der Siegel und Vermischen der einzelnen Komponenten erzeugte Konzentrat lässt sich schließlich in Wasser oder ein anderes Getränk einrühren und konsumieren.

Da die Reaktivdesorption in hohem Maße pH-Wert-abhängig ist, ist es oft von Vorteil, die Zusammenführung der einzelnen Komponenten in einem möglichst hohen Konzentrationsbereich vorzunehmen. Auch lässt sich die Löslichkeit der jeweiligen Nanopartikel irreversibel schädigen, wenn intermediär ein ungünstiger pH-Wert vorliegt. Gerade das Zusammenmischen der konzentrierten Komponenten ermöglicht ein schnelles und homogenes Durchlaufen des isoelektrischen Punktes und wirkt der Passivierung durch das Ausfallen von amphoteren Spezies im neutralen pH-Bereich entgegen. Bezogen auf mögliche Einrichtungen, in der die Speicherung und das Zusammenbringen der einzelnen Komponenten erfolgen kann, wird auf den Anspruch 11 verwiesen. Damit ist es möglich, dass sich eine gezielte Mischungssequenz einstellt. Besonders bevorzugt ist eine Vorrichtung, die die einzelnen Komponenten im Verschlusssystem einer Flasche integriert, um somit leicht die vollständige und sequentiell richtige Zubereitung des fertigen Vitalstoffgetränks in einem abgeschlossenen System zu ermöglichen.

Eine Einrichtung zur Herstellung von nanoskalisch wirkstoff-/vitalstoffenthaltenden Flüssigkeiten und Getränken entsprechend dem dargelegten Verfahren im Sinne einer Konfektionierung ist durch folgende Merkmale gekennzeichnet. Die Einrichtung enthält:
i. ein Reservoir 1 mit einer Komponente A,
ii. ein Reservoir 2 mit einer Komponente B,
iii. ein Trennsegment zwischen Reservoir 1 und Reservoir 2, und
iv. ein Gewinde, mit dem es auf eine Flasche geschraubt werden kann.

Die Einrichtung gemäß der Erfindung enthält insbesondere
i. ein erstes Reservoir, in welchem eine erste Komponente bereitgestellt werden kann,
ii. ein zweites Reservoir, in welchem eine zweite Komponente bereitgestellt werden kann,
iii. ein erstes lösbares Trennsegment zwischen dem ersten und dem zweiten Reservoir, und
iv. eine Ausbildung, um die Reservoire mit einem Behältnis für eine Flüssigkeit zu verbinden, wobei ein zweites lösbares Trennsegment ausgebildet ist, so dass das Reservoir geöffnet wird, wenn das zweite Trennsegment gelöst wird, so dass der Inhalt des ersten und/oder des zweiten Reservoirs freigegeben wird.

Die Ausbildung, um die Einrichtung mit einem Behältnis für eine Flüssigkeit zu verbinden, kann ein Gewinde umfassen.

Das Behältnis für eine Flüssigkeit kann beispielsweise eine Flasche sein. Als Flüssigkeit kommt insbesondere Wasser in Frage. Das zweite Trennsegment ist im Rahmen einer bevorzugten Weiterbildung als Berstscheibe, insbesondere als asymmetrische Berstscheibe, ausgebildet.

Mit Hilfe dieser Ausbildung der Einrichtung lassen sich die Reaktivkomponenten geeignet zusammenbringen. Damit wird dem Anwender die Möglichkeit gegeben, die a priori instabilen nanoskalischen Wirkstoff- bzw. Vitalstoffspezies unmittelbar vor der Einnahme/Applikation gesteuert freizusetzen. Die Einrichtung eignet sich insbesondere allgemein zur Darstellung von Flüssigkeiten und Getränken, deren Komponenten in einer speziellen Sequenz vermischt werden müssen, um eine optimale Löslichkeit zu erzielen.

Durch äußere Krafteinwirkung, zum Beispiel durch eine Dreh-, Biege-, Zieh- oder Drückbewegung am äußeren Verschluss, öffnet das innere Trennsegment zwischen Reservoir 1 und Reservoir 2 und die beiden Komponenten werden in Kontakt gebracht. Mit Hilfe des Gewindes stellt die Einrichtung einen Flaschenverschluss dar, der bequem an- und abgeschraubt werden kann. Vorteilhaft ist ferner, dass in mindestens einem Reservoir ein Überdruck vorgelegt wird, mit dessen Hilfe ein zweites Trennsegment, das zum Beispiel den Verschlussinhalt hin zu dem Inhalt einer Flasche abtrennt, über ein mit Druck beaufschlagbares Medium, vorzugsweise pneumatisch, in einer zweiten Stufe geöffnet werden kann. Diese Option lässt sich vorteilhaft mittels einer volumenflexiblen Konstruktion des Reservoirs 2, das sich auf einem geringeren Druckniveau als Reservoir 1 befindet, umsetzen. Hierzu bringt man bevorzugt die mindestens zwei Komponenten in einen Ziehharmonikaverschluss ein, der bevorzugt aus Kunststoff gefertigt wird.

Die Erfindung stellt bei ihrer Realisierung ein Nahrungsergänzungsmittel und/oder Erfrischungsgetränk zur Verfügung, welches nach dem erfindungsgemäßen Verfahren herstellbar ist, wobei das Nahrungsergänzungsmittel und/oder Erfrischungsgetränk kolloidal gelöste organische und mineralische Nanopartikel enthält.

Zudem stellt die Erfindung nanoskalische Pharmazeutika zur Verfügung, welche mit dem erfindungsgemäßen Verfahren herstellbar sind und kolloidal gelöste organische und mineralische Nanopartikel enthalten.

Eine wichtige Anwendung des erfindungsgemäßen Verfahrens ist dessen Nutzung innerhalb einer Zahnpaste zur Bereitstellung hochgradig bioverfügbarer Vitalstoffcremes, insbesondere von Mineralstoffsystemen. Als Aktivkomponente, die mit in den Zahnschmelz eingebaut werden muss, um diesem eine hohe Widerstandskraft zu geben, werden hier neben dem Kalzium und Magnesium der Speichermatrix vorzugsweise Strontium, Bor, Mangan, Silizium, Zink und Kupfer in die Speichermatrix eingebracht.

Vorteilhaft wird die saure Reaktivkomponente in ein Gel eingearbeitet, beispielsweise auf Basis von SiO₂-Flocken und/oder Laponiten. Eine diesbezügliche Zahnpastatube umfasst 3 Kammern, in denen ein saurer, ein basischer und ein außen umgebender Zahnpastastrang enthalten sind. Die Reaktiv-desorption erfolgt dann in situ beim Zähneputzen. Damit werden die entsprechenden hochgradig bioverfügbaren Mineral- und/oder Vitalstoffe genau zur richtigen Zeit am richtigen Ort freigesetzt.

Ähnlich lassen sich hochgradig vitalisierende Cremes herstellen, wobei die Säure einem Verdickungsmittel beispielsweise SiO₂, Laponite und/oder Stärke zugesetzt wird. Wiederum mit einer Drei-Strang-Tube lässt sich das System mit einer Cremephase kombinieren. Beim Verreiben in den Händen oder am Körper werden die hochgradig bioverfügbaren Vitalstoffe freigesetzt und können so optimal von der Haut aufgenommen werden. Analoge Creme-Systeme für den Kosmetikbereich, aber auch als Zahnpasta, können über 3-Komponentenbeutel bzw. Sticks eingebracht werden - hier hat man den Vorteil, dass die Dosierung, insbesondere die Abstimmung der 3 Komponenten, einfacher zu handhaben ist.

Eine sehr bedeutende Anwendung der nach dem NRD-Verfahren dargestellten Vitalstoffkonzentrate und Lösungen ist die hiermit mögliche Anreicherung von Lebensmitteln.

Vorteilhaft ist dabei, wenn das jeweilige Lebensmittel eine hohe Oberfläche und/oder ein hohes Saugvermögen besitzt. Diese Eigenschaften unterstützen eine homogene Verteilung der Vitalstoffe, die bevorzugt im nanoskalischen Bereich angeboten werden, da hier der Sorptionsprozess schnell gegenüber dem Agglomerisationsprozess erfolgt. Sehr gut geeignete Lebensmittel sind ferner Kakaopulver, Cerealien, Milchpulver, Zucker. Sehr gut geeignet sind in diesem Kontext auch Mehle und Getreidegrieß.

Besonders vorteilhaft ist auch die Herstellung von Dentalkaugummi, wobei tiefgekühltes gemahlenes Kaugummipulver - z. B. mit flüssigem N2 versprödetem Kaugummi - im Vakuumgefriertrockenprozess mit dem Vitalstoffkonzentrat besprüht und entsprechend beladen wird. Ein derartiger besonders mit Ca-, Mg-, Sr-, B-beladener Kaugummi führt zu einer optimalen Versorgung der Zähne mit bioverfügbaren Mineralien.

Ferner eignen sich immobilisierende Lebensmittel sehr gut, um die hochenergetischen Vitalstoff-Partikel auf Abstand zu halten. Diese sind Gelatine-Produkte, Pektin-Produkte oder auch Teig-Produkte. Insbesondere Marmelade, Konfitüre, Fruchtgummi-Produkte, Schokolade und Schokoladencreme-Produkte. Ebenso lässt sich auch Tierfutter mit der erfindungsgemäßen Vitalstoffdispersion herstellen.

Die Erfindung wird nachfolgend anhand von Ausführungsbeispielen unter Bezugnahme auf die beigefügten Figuren näher erläutert. Dieselben Bauteile sind in den Figuren mit denselben Bezugszeichen versehen. In der zugehörigen Zeichnung zeigt
- Fig. 1: Spektrum der Partikelverteilung entsprechend Beispiel 1,
- Fig. 2: Querschnitt des Dreikammerverschlusses/Modus "Geschlossen",
- Fig. 3: Schnitt A von Fig. 2/Aufsicht,
- Fig. 4: Schnitt B von Fig. 2/Aufsicht,
- Fig. 5: Schnitt C von Fig. 2/Aufsicht,
- Fig. 6: Schnitt D von Fig. 2/Aufsicht,
- Fig. 7: Querschnitt des Dreikammerverschlusses/Modus "Aktiviert",
- Fig. 8: Querschnitt des Dreikammerverschlusses/Modus "Geöffnet",
- Fig. 9: fixierte Doppelkammerkartusche im Inneren einer Getränkeflasche, die mit Hilfe einer Fixiervorrichtung gehalten wird,
- Fig. 10: schematische Darstellung der Öffnungssequenz der Doppelkammerkartusche, die pneumatisch initiiert wird und sich wiederum pneumatisch fortpflanzt, und
- Fig. 11: Beispiel einer Messung einer Partikelgrößenverteilung einer thixotropen Speichermatrix.

### Ausführungsbeispiel 1

500 g Leinsamenpresskuchen, der zuvor mittels Hexanextraktion auf einen Restölgehalt von ca. 1 % entölt wurde, wird in einem 3 I-Rundkolben mit 3 x 1, 5 I Aceton, dem jeweils 100 mg Tocopherol zugesetzt wurde, bei 40°C im Ultraschallbad, jeweils 20 Minuten extrahiert.

Die Extraktfraktionen wurden vereinigt, mit 25 g Zitronensäure versetzt und im Rotationsverdampfer im Wasserbad bei einem Druck von ca. 100 mbar zur Trocknung eingedampft. Der Rückstand wurde aus dem Rundkolben herausgekratzt und in einer Kugelmühle 5 Stunden gemahlen.

Das gemahlene Pulver wurde wiederum im Rotationsverdampfer bei 60°C bei einem Druck < 5 mbar für eine Stunde ausgedampft und ergab somit das organisch-saure sorptive System.

Ein entsprechend extrahierter Leinsamenpresskuchenrückstand von 5 kg wurde in Tiegeln bei ca. 1.000°C verbrannt, die Asche zusammengetragen, auf 50 µm abgesiebt und in einem Tiegel für 48 h in einem Muffelofen bei 1.150°C erhitzt.

Die somit hergestellte hochreine Asche wurde mit 200 ml Wasser in einer rotierenden Mahlperlenmühle mit Mahlkörpern der Größe 1,5 bis 2,5 mm vorgemahlen mit einem Energieeintrag von 0,5 kWh/kg bezogen auf den Feststoff und in einer zweiten Mahlstufe mit Mahlperlen von 0,3 - 0,4 mm und einem Energieeintrag von 3 kWh/kg - feststoffbezogen - gemahlen.

Der Gehalt an Kalzium- und Magnesiumcarbonaten beziehungsweise Hydroxiden war dabei ca. 40 Gew. % bezogen auf die Trockenmasse, beziehungsweise ca. 5 Gew. % bezogen auf die Suspension. Der pH-Wert war größer als 11 und betrug 13,8.

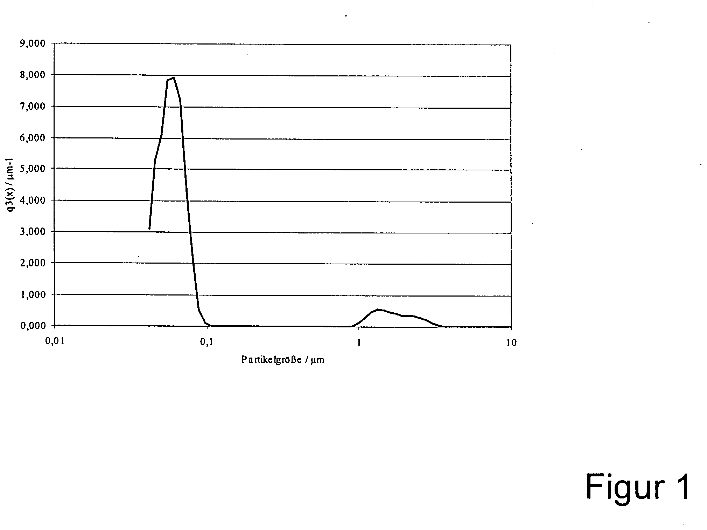

2,0 g der hierbei hergestellten basischen Suspension wurden mit 1 g des organisch-sauren sorptiven Systems auf einen pH-Wert zwischen 3 und 4 eingestellt und mit 150 ml Wasser verdünnt. Die sich hieraus ergebende kolloidale Lösung wurde anhand von Beugungsmessungen charakterisiert und dessen nanoskalischer Charakter bestätigt (siehe Fig. 1).

Die Messung wurde anhand der Laser-Beugungs-Spektroskopie nach der PIDS-Technik bestimmt (Polarisation Intensity Differential Scattering) mit einem Spektrometer des Typus LS230 der Firma Beckmann-Coulter GmbH/Krefeld, wobei das Partikelspektrum entsprechend der Volumendichteverteilung q3 berechnet und aufgetragen wurde (siehe: H. Rumpf, "Mechanische Verfahrenstechnik", Carl Hanser Verlag/München-Wien 1995, S. 12 -15).

### Ausführungsbeispiel 2

Es wurden 400 g Kalziumhydroxid, 200 g Magnesiumoxid nebst 50 g KOH als Flussmittel 12 h in einem Korundtiegel bei 1.100°C gesintert. Das Sinterprodukt wurde in einer Kugelmühle vorgemahlen und in einer Perlmühle wässrig, zusammen mit 1.000 mg Eisen(III)oxid und 100 mg Silberoxid, fein gemahlen. Der schlussendliche Feststoffgehalt der thixotropen Suspension lag bei 19,5 %. Das Partikelverteilungsspektrum hatte ein Maximum zwischen 300 und 500 nm; nahezu 90 % aller Partikel waren kleiner 1 µm.

Mit Hilfe eines Rotationsviskosimeters der Firma Mittler Toledo wurde eine Viskosität von 6.000 mPa·s und eine Thixotropie von 1.200.000 mPa·s bestimmt. Die Dichte betrug 1,12 g/cm³. 5 g dieser Suspension wurden in einem Schnappdeckelgläschen mit 1,5 g Zitronensäure versetzt und leicht geschüttelt. Das entstandene Konzentrat wurde mit destilliertem Wasser 40-fach verdünnt und die Lösung bzw. nanoskalische Dispersion 10 Minuten nach deren Herstellung bzw. Aktivierung entsprechend dem NRD-Verfahren wiederum per Laser-Beugungsspektroskopie vermessen.

Das Resultat war ein Maximum der Partikelverteilung bei 60 nm, wobei nahezu 100 % aller Partikel kleiner 100 nm waren.

Der Wiederholungsversuch mit der gleichen Suspension 10 Tage später zeigte das gleiche Resultat.

Die Dispersion wurde nach 2 h nochmals vermessen und zeigte dann ein Maximum der Partikelverteilung bei 250 nm, nach weiteren 4 h setzte sich ein erkennbarer weißlich-gelber Niederschlag ab, der aufgerührt werden musste und dann ein Maximum der Partikelverteilung bei 1,2 µm ergab.

### Ausführungsbeispiel 3

5 g der im Ausführungsbeispiel 2 hergestellten basisch-mineralischen thixotropen Speichermatrix wurden mit 1,25 g des organisch-sauren sorptiven Systems aus Ausführungsbeispiel 1 in einem 10 ml Schnappdeckelgläschen kontaktiert und kurz geschüttelt. Sofort wurde das Gesamtsystem dünnflüssig und bildete eine nanoskalisch-kolloidale Dispersion. Diese wurde wiederum 40-fach verdünnt und 10 Minuten nach Aktivierung beziehungsweise Durchführung des reaktiven Schrittes des NRD-Verfahrens über Laserbeugung vermessen.

Das Maximum der relativ steil verlaufenden Partikelverteilung lag bei ca. 75 nm, wiederum waren nahezu 100 % aller Spezies kleiner 100 nm.

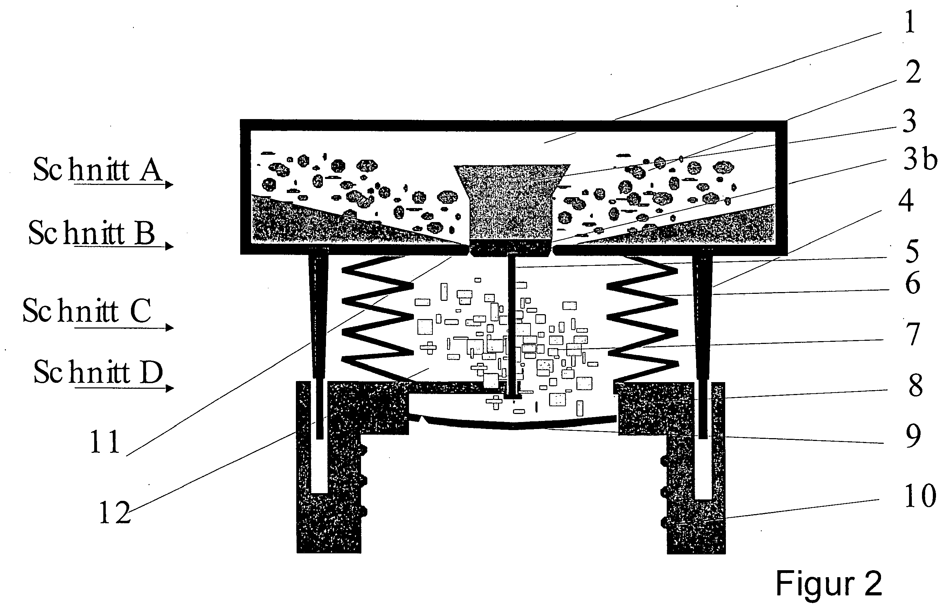

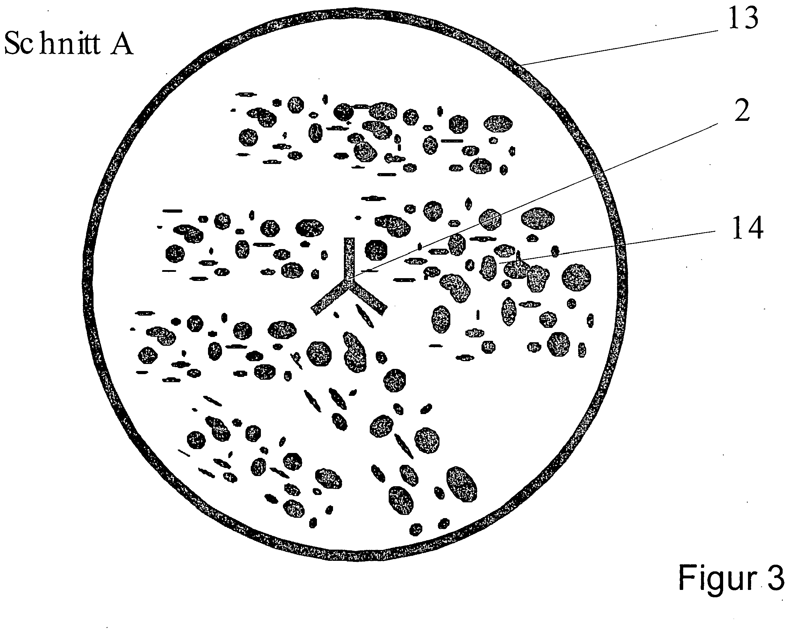

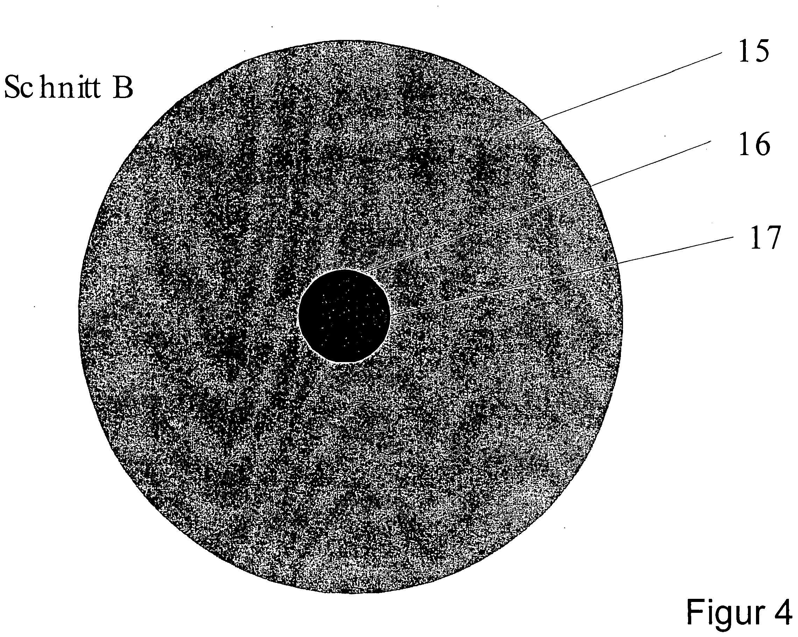

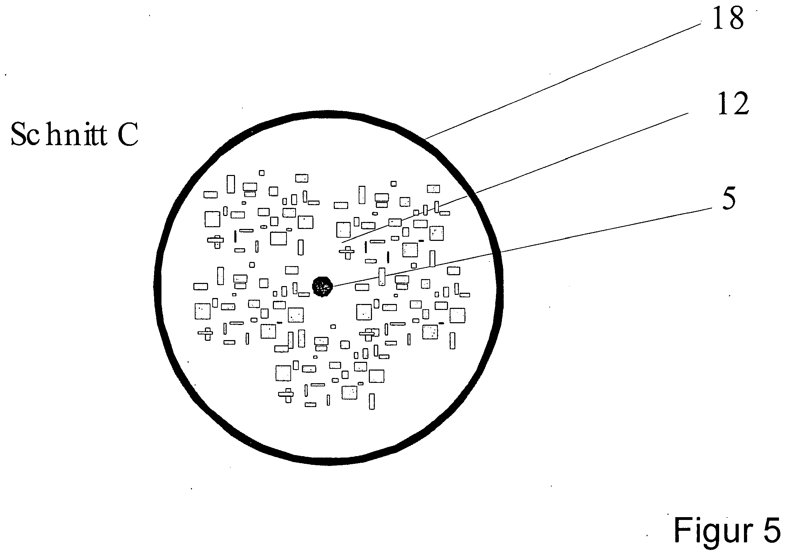

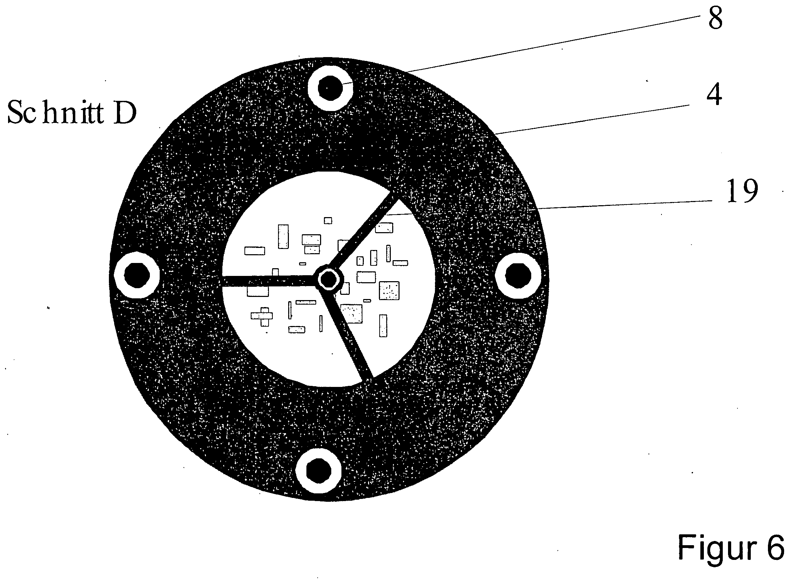

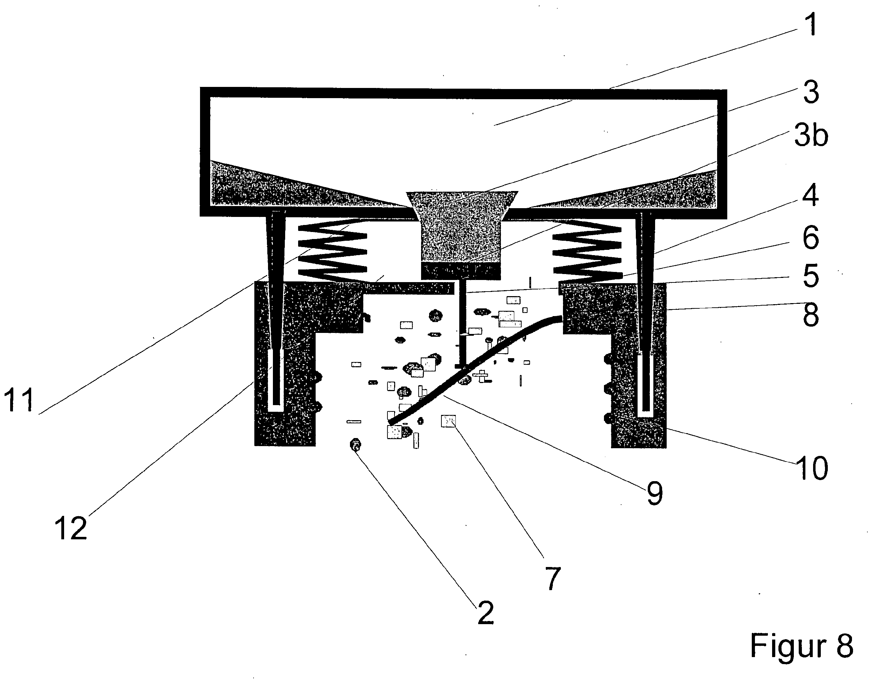

Im Folgenden wird die Einrichtung zur Konfektionierung der nach dem erfindungsgemäßen Verfahren hergestellten Komponenten anhand der Fig. 2 bis 10 beschrieben. Die Einrichtung ist besonders bevorzugt derart gestaltet, dass in mindestens einem Reservoir ein Überdruck vorgelegt wird, mit dessen Hilfe ein zweites Trennsegment, das zum Beispiel den Verschlussinhalt hin zu dem Inhalt einer Flasche abtrennt, über ein mit Druck beaufschlagbares Medium, beispielhaft pneumatisch, in einer zweiten Stufe geöffnet werden kann. Diese Option lässt sich vorteilhaft mittels einer volumenflexiblen Konstruktion des Reservoirs 2, das sich auf einem geringeren Druckniveau als Reservoir 1 befindet, umsetzen. Hierzu bringt man bevorzugt die mindestens zwei Komponenten in einen Ziehharmonikaverschluss ein, der bevorzugt aus Kunststoff gefertigt wird und exemplarisch in Fig. 2 bis 8 dargestellt ist.

Die Fig. 2 bis 8 illustrieren einen Ziehharmonikaverschluss der erfindungsgemäßen Vorrichtung. Die Komponente I - vorzugsweise das sorptive System 2 - wird im oberen Verschlussdeckel vorgelegt. Vorzugsweise wird der Verschlussdeckel dabei unter einen Überdruck von bis 30 bar gesetzt, der dann die Öffnung der Vorrichtung unterstützt. Typischerweise besteht eine Druckdifferenz zwischen den beiden Reservoirs zwischen 0,5 und 20 bar. Der Verschlussdeckel ist zunächst geschlossen, wobei der Pfropfen 3/3b, insbesondere der Verschlussbereich des Pfropfens 3b, die Komponente I 2 und die Komponente II 7, die im Reservoir II 12 vorgelegt wird, voneinander trennt.

Aktiviert wird der Verschluss entweder durch eine Dreh- und/oder Biegebewegung am Verschlussdeckel, wodurch die Siegelzone 11, die den Verschlussbereich des Pfropfens, der über dem Befestigungsstab 5 fixiert ist, umgibt, bricht. Die Expansion des Überdrucks im Verschlussdeckel stößt dann den Pfropfen nach unten, der dann über den Öffnungsbereich des Pfropfens den Druckausgleich und die Durchmischung der beiden Komponenten ermöglicht. Das Reservoir 2, das durch den Ziehharmonikaschlauch 6 umgeben wird, expandiert durch die Druckarbeit impulsartig, wodurch der fixierte Pfropfen in der geöffneten Position durch das sich konisch ausweitende sternförmige Zackengerüst in der Öffnung des Bodens des oberen Verschlussdeckels verhakt wird. Diese Volumenflexibilität ist insbesondere einer sicheren Verwendung der erfindungsgemäßen Vorrichtung sehr zuträglich.

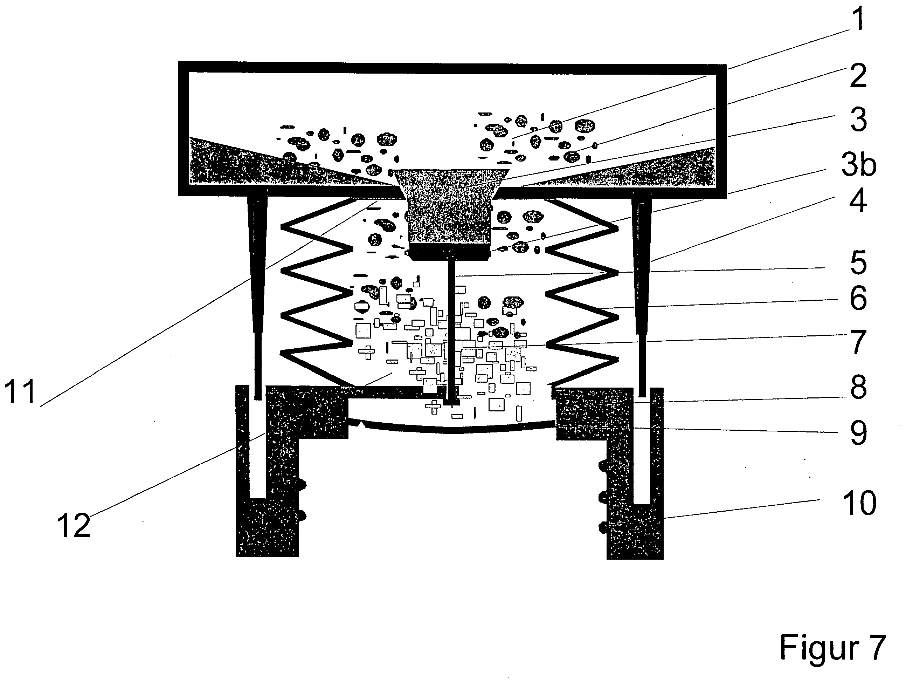

In dem aktivierten Zustand, der in Fig. 7 gezeigt ist, liegt im Bereich innerhalb des Ziehharmonikaschlauches eine genügend große Gasmenge vor, die insbesondere noch anhand einer optionalen Freisetzung von Kohlendioxid im Rah men der Reaktivdesorption erhöht wird, um im eigentlichen Öffnungsschritt durch manuellen Druck auf den Verschlussdeckel und der damit erzielten Kompression/Druckerhöhung die asymmetrische Berstscheibe 9 zum Platzen bringt. Die geöffnete Berstscheibe 9 wird durch den nach unten gedrückten Fixierstab des Pfropfens schließlich in Offenstellung gehalten. Die Fixierstäbe werden durch die absenkende Bewegung des oberen Verschlussdeckels, an dem sie fixiert sind, mit ihrer konusförmigen Ausprägung in die zylindrischen Aussparungen in den unteren Verschlussdeckel getrieben und wiederverhakt. Mit dem damit hergestellten festen Kontakt der beiden Verschlusshälften kann der im gesamten Prozess am Flaschenkopf befestigte Deckel nach dem Durchschütteln und Vermischung des in die Flasche eingeflossenen nanoskalischen Konzentrats aufgeschraubt werden.

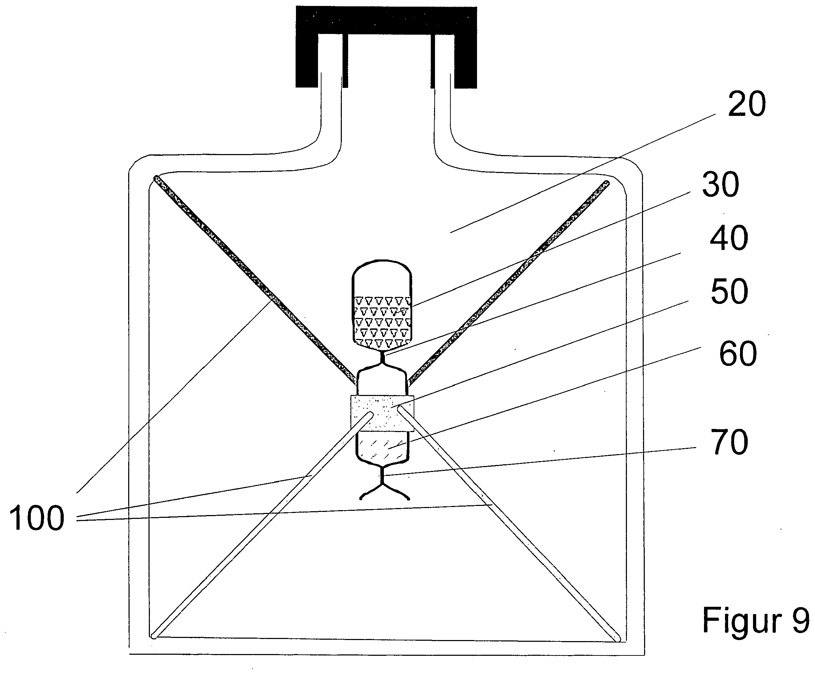

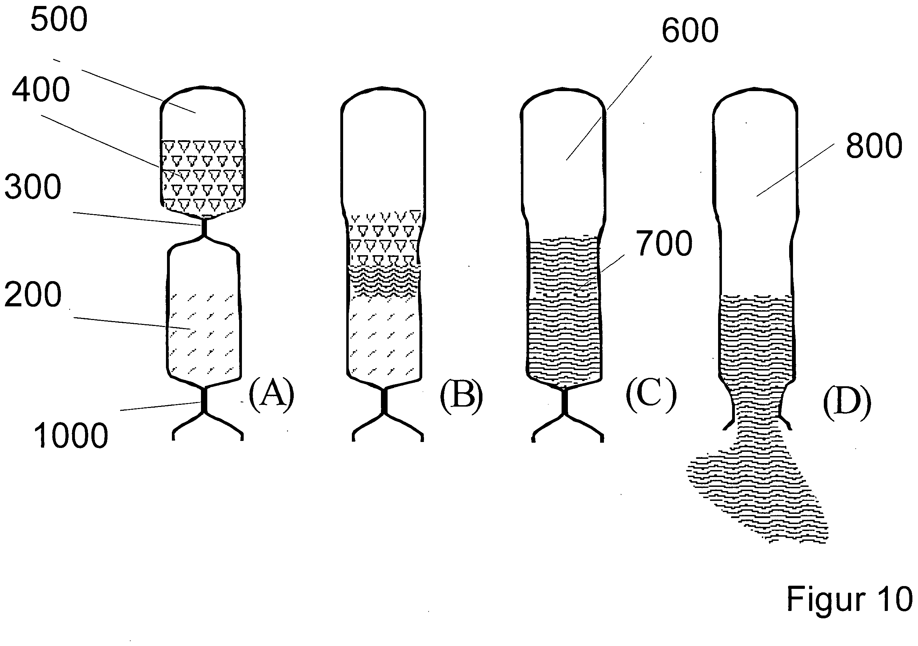

Fig. 9 zeigt eine fixierte Doppelkammerkartusche im Inneren einer Getränkeflasche, die mit Hilfe einer Fixiervorrichtung gehalten wird. Das Siegel 40 öffnet konsekutiv, nachdem die Flasche kurz geöffnet wird und der sich in der Flasche befindliche Überdruck entspannen kann. Fig. 10 zeigt die Öffnungssequenz einer Doppelkammerkartusche der erfindungsgemäßen Einrichtung, die pneumatisch initiiert wird und sich wiederum pneumatisch fortpflanzt.

Fig. 10 zeigt den Querschnitt der Doppelkammerkartusche zu Beginn des Öffnens. Bild B zeigt die Kartusche, nachdem das Siegel 300 geöffnet und die Komponenten in Kammer 400 auf die Komponenten in die untere Kammer gerieselt sind. Vorausgegangen ist das Öffnen der äußeren Flasche, die zuvor unter erhöhtem Druck gestanden hat. Dieser erhöhte Druck hat ausgereicht, um die Kartusche im deformierten Zustand gemäß Bild A zu halten.

Bild C zeigt die konzentrierte Dispersion 700, nachdem die Flasche nebst Kartusche geschüttelt wurde. Die konzentrierte Dispersion 700 hat sich auf Basis der nanoskalischen Reaktiv-Desorption gebildet.

Wie in Bild D dargestellt, hat sich auf Basis des sich vollziehenden Druckanstiegs das untere beziehungsweise das zweite Siegel geöffnet. Der sich entspannende Überdruck drückt den jetzt dünnflüssigen Kartuscheninhalt in die umgebende Flaschenflüssigkeit.

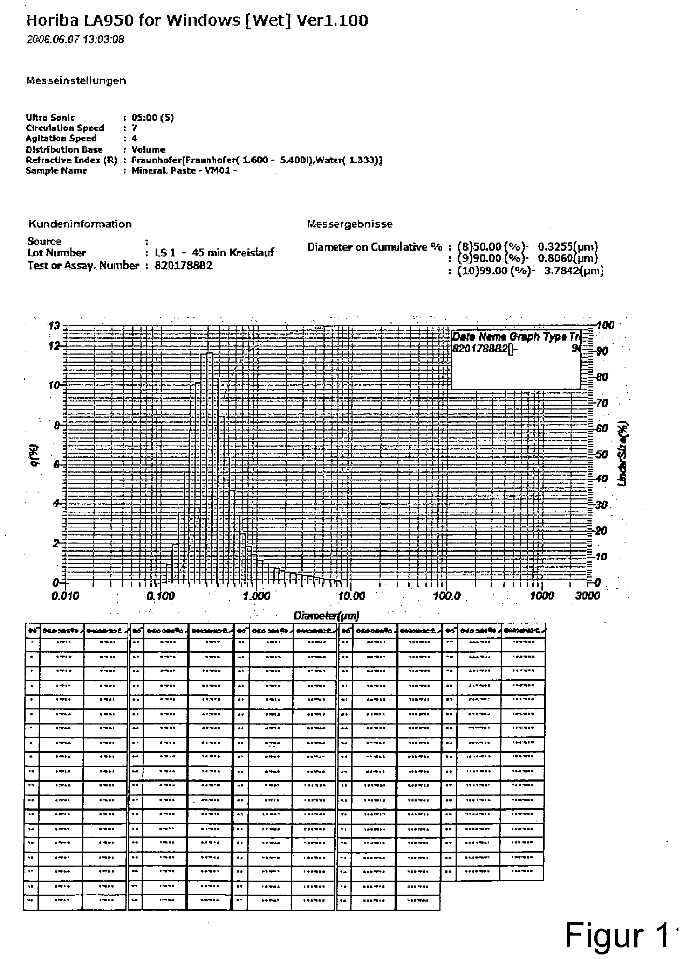

In Fig. 11 ist beispielhaft eine Partikelgrößenverteilung für das sorptive System gezeigt, welches als thixotrope Speichermatrix, das mit dem oben beschriebenen Verfahren zur Herstellung einer strukturviskosen, insbesondere einer thixotropen und säurelöslichen Speichermatrix, insbesondere zur Verwendung als sorptive und/oder immobilisierende Feststoffmatrix im Verfahren zur Solubilisierung von Vitalstoffen erzeugt werden kann.

### Bezugszeichenliste

- 1: Oberer Verschlussdeckel/Reservoir 1 - vorzugsweise unter Überdruck
- 2: Komponente 1
- 3: Öffnungsbereich des Pfropfens mit Widerverhakung
- 3b: Verschlussbereich des Pfropfens
- 4: Fixierstab mit kegelförmiger Widerverhakung
- 5: Befestigungsstab für Pfropfen, der zugleich die Berstscheibe offen hält
- 6: Ziehharmonikaschlauch
- 7: Komponente II
- 8: Primärverschluss
- 9: Asymmetrische Berstscheibe
- 10: Schraubverschluss zum Anschrauben an Flaschenöffnung
- 11: Siegelzone, die durch Dreh- oder Kippbewegung am oberen Verschlussdeckel geöffnet wird
- 12: Reservoir 2
- 13: Äußere Wandung des oberen Verschlussdeckels
- 14: Komponente 1
- 15: Unterseite oberer Verschlussdeckel
- 16: Versiegelungszone
- 17: Verschlussbereich des Pfropfens
- 18: Ziehharmonikaschlauch
- 19: Fixiersteg für Pfropfenbefestigung
- 20: Flascheninneres
- 30: Obere Kartuschenkammer, die mit einer Reaktionskomponente befüllt ist, vorzugsweise mit einem trockenen, nicht-wässrigen System
- 40: Versiegelter Bereich, der pneumatisch geöffnet werden kann und dann die Bereiche 30 und 60 verbindet
- 50: Halterung des Fixierelements
- 60: Untere Kartuschenkammer, die mit der 2. Reaktionskomponente befüllt ist, vorzugsweise mit einer wässrig-basischen Komponente
- 70: 2. Siegel, das durch den Überdruck öffnet, der sich auf Basis der Reaktion der beiden Komponenten ergibt, die von einer Gasentwicklung begleitet ist
- 100: Fixierelement zur Halterung der Kartusche innerhalb der Flasche
- 200: Untere Kartuschenkammer, die mit der 2. Reaktionskomponente befüllt ist, vorzugsweise mit einer wässrig-basischen Komponente
- 300: Versiegelter Bereich, der pneumatisch geöffnet werden kann und dann die Bereiche 200 und 400 verbindet
- 400: Obere Kartuschenkammer, die mit einer Reaktionskomponente befüllt ist, vorzugsweise mit einem trockenen, nicht-wässrigen System
- 500: Unter Druck stehendes Gasvolumen - größer 1,5 bar
- 600: Bereich
- 700: Konzentrierte Dispersion
- 1000: 2. Siegel, das durch den Überdruck öffnet, der sich auf Basis der Reaktion der beiden Komponenten ergibt, die von einer Gasentwicklung begleitet ist

## Patentansprüche

1. Verfahren zur Solubilisierung von in Wasser im pH-Bereich 3 bis 7 schwerlöslichen mineralischen Wirkstoffen und deren Bereitstellung als bioverfügbares Konzentrat, das Verfahren umfassend:
a) Herstellen einer wässrigen Basissuspension der mineralischen Wirkstoffe;
b) Vermahlen der Basissuspension mit einem basischen Feststoff ausgewählt aus der Gruppe Kalziumhydroxid, Kalziumcarbonat, Magnesiumhydroxid, Magnesiumcarbonat und Mischungen davon als Matrix, wobei die Wirkstoffe von der basischen-mineralischen FeststoffMatrix sorbiert werden und mit ihr ein sorptives System bilden, wobei der Massenanteil der Wirkstoffe bezogen auf die Summe aus Fest- und Wirkstoffen 0,05 %-30 % beträgt, und
c) Vermischen des so gebildeten wasserhaltigen sorptiven Systems mit einer sauren Reaktivkomponente ausgewählt aus festen organischen Säuren,
so dass
i) die basisch-mineralische Feststoffmatrix durch eine Säure-Base-Reaktion aufgelöst wird und dabei
ii) die von der basisch-mineralischen Feststoffmatrix sorbierten Wirkstoffe im Verlauf einer Reaktivdesorption als kolloidal in Wasser gelöste Nanopartikel freigesetzt werden und somit das bioverfügbare Konzentrat darstellen,
**dadurch gekennzeichnet, dass** das sorptive System und die saure Reaktiv-Komponente in Schritt c) mithilfe eines Mehrkammerbehältnisses zusammengebracht werden, und dadurch, dass der basische Feststoff aus Holzasche und/oder Kohleasche erhalten wird.

2. Verfahren nach Anspruch 1, wobei die freigesetzten kolloidal gelösten Nanopartikel in der Zeitspanne unmittelbar nach der Reaktivdesorption und Verdünnen auf eine trinkfertige Konzentration ein Partikelwachstum, bezogen auf den anfänglichen mittleren Partikeldurchmesser, von mindestens 5 %, typischerweise mindestens 50 % pro Stunde zeigen.

3. Verfahren nach einem der vorgenannten Ansprüche, wobei zwischen Schritt b) und Schritt c) mindestens 10 Tage liegen.

4. Verfahren nach einem der vorgenannten Ansprüche, wobei die wässrige Suspension einen pH-Wert von >10 aufweist.

5. Verfahren nach einem der vorgenannten Ansprüche, wobei die saure Komponente im Wesentlichen aus Zitronensäure besteht.

6. Verfahren nach einem der vorgenannten Ansprüche, wobei die sorbierten Spezies Spuren- bzw. Ultraspurenelemente, umfassend Silizium, Eisen, Aluminium, Mangan, Chrom, Bor, Titan, Nickel, Kupfer, Zink, Vanadium, Molybdän, Kobalt, Selen, Zirkon, Strontium, Indium, Rubidium, Lithium, Yttrium, Cer, Palladium, Lanthan, Neodym, Silber, Wolfram, Gallium, Tellur, Thorium, Praseodym, Niob, Samarium, Gadolinium, Dysprosium, Arsen, Scandium, Indium, Antimon, Cäsium, Germanium, Ytterbium, Erbium, Europium, Bismut, Platin, Tantal, Terbium, Holmium, Beryllium, Gold, Strontium und/oder Rhodium enthalten.

7. Verfahren nach Anspruch 6, wobei das Verhältnis zwischen Spuren- und Ultraspurenelementen einerseits und der Summe Kalzium plus Magnesium plus Kalium andererseits zwischen 0,1 und 20 Gew.-%, bevorzugt zwischen 0,3 und 10 Gew.-% und besonders bevorzugt zwischen 0,5 und 5 Gew.-% liegt.

8. Verfahren nach einem der vorgenannten Ansprüche, ferner umfassend, nach Abschluss des Verfahrensschritts c), die Verwendung des Endprodukts zum Auf- und/oder Einbringen auf bzw. in sorbierende und/oder immobilisierende Lebensmittel, nämlich Getreideprodukte, insbesondere Mehl, Cerealien, Hartweizengrieß, Kakaopulver, Zucker, Milchpulver, Stärke, oder gelatinehaltige und/oder pektinhaltige Lebensmittel wie Marmelade, Konfitüre oder Fruchtgummis.

9. Verfahren zur Herstellung eines mit Wirkstoffen angereicherten Lebensmittels, umfassend das Verfahren nach Anspruch 8, wobei das bioverfügbare Konzentrat nach seiner Herstellung innerhalb von weniger als 5 Stunden, weniger als 1 Stunde oder weniger als 5 Minuten nach der Durchführung von Schritt c) auf bzw. in das Lebensmittel auf- und/oder eingebracht wird.

10. Verfahren nach einem der vorgenannten Ansprüche, wobei das sorptive System und die saure Reaktiv-Komponente in Schritt c) mithilfe einer Mehrkammertube zusammengebracht werden.

11. Vorrichtung zur Herstellung eines Wirkstoffpräparats, welches nach dem Verfahren nach Anspruch 1 oder einem der davon abhängigen Ansprüche erhältlich ist, wobei die Vorrichtung aus einem Beutel besteht, in dem zwei Kammern voneinander durch ein Siegel separiert sind, wobei die eine Kammer das basisch-mineralische sorptive System enthält und die andere Kammer die saure Reaktivkomponente aus festen organischen Säuren enthält, wobei das Siegel dazu ausgebildet ist, sich auf Druck auf eine der Kammern zu öffnen und somit das basisch-mineralische sorptive-System und die saure Reaktiv-komponente aus festen organischen Säuren miteinander in Kontakt zu bringen.

12. Verwendung einer nach dem Verfahren eines der Ansprüche 1 bis 7 hergestellten Wirkstoffdispersion als Nahrungsergänzungsmittel oder Erfrischungsgetränk.

13. Verwendung einer nach dem Verfahren eines der Ansprüche 1 bis 7 hergestellten Wirkstoffdispersion als Zusatz zu pharmazeutischen Präparaten.

14. Verwendung einer nach dem Verfahren eines der Ansprüche 1 bis 7 hergestellten Wirkstoffdispersion als Zusatz zu kosmetischen Präparaten.

15. Verwendung nach Anspruch 14, wobei das kosmetische Präparat eine Creme ist.

16. Verwendung einer nach dem Verfahren eines der Ansprüche 1 bis 7 hergestellten Wirkstoffdispersion als Zusatz zu einer Zahnpasta.

## Claims

1. A method of solubilising mineral agents poorly soluble in water in a pH range of 3 to 7, and of providing same as a bioavailable concentrate, the method including:
a) preparing an aqueous base suspension of the mineral agents;
b) grinding the base suspension with a basic solid selected from the group calcium hydroxide, calcium carbonate, magnesium hydroxide, magnesium carbonate and mixtures thereof as a matrix, wherein the agents are adsorbed by the basic, solid mineral matrix and together with it form a sorptive system, wherein the mass content of the agents in relation to the sum of solids and agents is 0.05 % to 30 %, and
c) mixing the aqueous sorptive system so formed with an acidic reaction component selected from solid organic acids,
so that
i) the basic, solid mineral matrix is dissolved by an acid-base-reaction and thereby
ii) the agents adsorbed by the basic, solid mineral matrix are released in the course of a reactive desorption as nanoparticles dissolved in colloidal form in water and thus constitute the bioavailable concentrate,
**characterised in that** the sorptive system and the acidic reaction component are brought into contact in step c) by means of a multi-compartment container, and **in that** the basic solid is obtained from wood ash and/or coal ash.

2. The method of claim 1, wherein the released, colloidally dissolved nanoparticles undergo, in the time period directly after the reactive desorption and diluting to a drinkable concentration, a particle growth of at least 5 %, typically at least 50 % per hour, relative to the initial average particle diameter.

3. The method of one of the preceding claims, wherein there are at least 10 days between step b) and step c).

4. The method of one of the preceding claims, wherein the aqueous suspension has a pH value of >10.

5. The method of one of the preceding claims, wherein the acidic component consists essentially of citric acid.

6. The method of one of the preceding claims, wherein the adsorbed species include trace and ultra-trace elements, including silicon, iron, aluminum, manganese, chromium, boron, titanium, nickel, copper, zinc, vanadium, molybdenum, cobalt, selenium, zirconium, strontium, indium, rubidium, lithium, yttrium, cerium, palladium, lanthanum, neodymium, silver, tungsten, gallium, tellurium, thorium, praseodymium, niobium, samarium, gadolinium, dysprosium, arsenic, scandium, indium, antimony, cesium, germanium, ytterbium, erbium, europium, bismuth, platinum, tantalum, terbium, holmium, beryllium, gold, strontium, and/or rhodium.

7. The method of claim 6, wherein the ratio between trace and ultra-trace elements on the one hand and the sum of calcium plus magnesium plus potassium on the other hand is between 0.1 and 20 % by weight, preferably between 0.3 and 10 % by weight and particularly preferred between 0.5 and 5 % by weight.

8. The method of one of the preceding claims, further including, after completion of step c), the use of the product for applying it on or into adsorbing and/or immobilizing foods, namely cereal products, in particular flour, cereals, durum wheat semolina, cocoa powder, sugar, milk powder, starch, or gelatin containing and/or pectin containing foods such as marmalade, preserve, or jelly beans.

9. A method of manufacturing food enriched in agents, including the method of claim 8, wherein the bioavailable concentrate is applied onto or into the food, after its preparation, within less than 5 hours, less than 1 hour, or less than 5 minutes after the completion of step c).

10. The method of one of the preceding claims, wherein das sorptive system and the acidic reaction component are brought together in step c) by means of a multi chamber collapsible tube.

11. A device for preparing an agent preparation, which is obtainable according to the method of claim 1 or one the claims dependent therefrom, wherein the device consists of a bag, in which two chambers are separated by a seal, wherein the one chamber contains the basic mineral sorptive system and the other chamber contains the acidic reaction component of solid organic acids, wherein the seal is configured to break upon exertion of pressure onto one of the chambers, and to thereby bring into contact the basic mineral sorptive system and the acidic reaction component of solid organic acids.

12. Use of an agent dispersion manufactured according to the method of one of claims 1 to 7 as a nutrient additive or a refreshment beverage.

13. Use of an agent dispersion manufactured according to the method of one of claims 1 to 7 as an additive to pharmaceutical preparations.

14. Use of an agent dispersion manufactured according to the method of one of claims 1 to 7 as an additive to cosmetic preparations.

15. The use of claim 14, wherein the cosmetic preparation is a cream.

16. Use of an agent dispersion manufactured according to the method of one of claims 1 to 7 as an additive to a toothpaste.

## Revendications

1. Procédé de solubilisation de principes actifs minéraux difficilement solubles dans l'eau dans la plage de pH de 3 à 7 et de leur mise à disposition sous forme de concentré biodisponible, le procédé comprenant :
a) la production d'une suspension de base aqueuse des principes actifs minéraux ;
b) le broyage de la suspension de base avec un solide basique sélectionné parmi le groupe comprenant l'hydroxyde de calcium, le carbonate de calcium, l'hydroxyde de magnésium, le carbonate de magnésium et des mélanges de ceux-ci comme matrice, dans lequel les principes actifs sont absorbés par la matrice de solide basique minéral et forment avec elle un système de sorption, dans lequel la fraction massique des principes actifs rapportée à la somme de solides et de principes actifs est de 0,05 % à 30 %, et
c) le mélange du système de sorption aqueux ainsi formé avec un composant réactif acide sélectionné parmi les acides organiques solides,
de sorte que
i) la matrice de solide basique minéral est dissoute par une réaction acide-base et qu'alors
ii) les principes actifs absorbés par la matrice de solide basique minéral au cours d'une désorption réactive sont libérés sous forme de nanoparticules dissoutes dans l'eau de manière colloïdale et représentent ainsi le concentré biodisponible,
**caractérisé en ce que** le système de sorption et le composant réactif acide sont réunis à l'étape c) à l'aide d'un récipient à chambres multiples, et **en ce que** le solide basique est obtenu à partir de cendre de bois et/ou de cendre de charbon.

2. Procédé selon la revendication 1, dans lequel les nanoparticules dissoutes de manière colloïdale libérées présentent, dans la période qui suit immédiatement la désorption réactive et la dilution en une concentration prête-à-boire, une croissance de particules, par rapport au diamètre initial moyen des particules, d'au moins 5 %, typiquement d'au moins 50 % par heure.

3. Procédé selon l'une des revendications précédentes, dans lequel au moins 10 jours séparent l'étape b) de l'étape c).

4. Procédé selon l'une des revendications précédentes, dans lequel la suspension aqueuse présente une valeur de pH de > 10.

5. Procédé selon l'une des revendications précédentes, dans lequel le composant acide se compose essentiellement d'acide citrique.

6. Procédé selon l'une des revendications précédentes, dans lequel les espèces absorbées contiennent des oligo-éléments ou ultra-oligo-éléments, comprenant le silicium, le fer, l'aluminium, le manganèse, le chrome, le bore, le titane, le nickel, le cuivre, le zinc, le vanadium, le molybdène, le cobalt, le sélénium, le zircon, le strontium, l'indium, le rubidium, le lithium, l'yttrium, le cérium, le palladium, le lanthane, le néodyme, l'argent, le wolfram, le gallium, le tellure, le thorium, le praséodyme, le niobium, le samarium, le gadolinium, le dysprosium, l'arsenic, le scandium, l'indium, l'antimoine, le césium, le germanium, l'ytterbium, l'erbium, l'europium, le bismuth, le platine, le tantale, le terbium, l'holmium, le béryllium, l'or, le strontium et/ou le rhodium.

7. Procédé selon la revendication 6, dans lequel le rapport entre les oligo-éléments et ultra-oligo-éléments d'une part et la somme du calcium plus magnésium plus potassium d'autre part est comprise entre 0,1 et 20 % en poids, de préférence entre 0,3 et 10 % en poids et de manière particulièrement préférée entre 0,5 et 5 % en poids.

8. Procédé selon l'une des revendications précédentes, comprenant en outre, au terme de l'étape de procédé c), l'utilisation du produit final en vue d'une application sur et/ou d'une introduction dans des aliments absorbants et/ou immobilisants, à savoir des produits céréaliers, en particulier de la farine, des céréales, de la semoule de blé dur, du cacao en poudre, du sucre, du lait en poudre, de l'amidon, des aliments à base de gélatine et/ou de pectine, tels que de la confiture, de la marmelade ou des gommes aux fruits.

9. Procédé de fabrication d'un aliment enrichi en principes actifs, comprenant le procédé selon la revendication 8, dans lequel le concentré biodisponible, après sa fabrication, est appliqué sur ou introduit dans l'aliment dans un délai de moins de 5 heures, de moins d'une 1 heure ou de moins de 5 minutes après la réalisation de l'étape c).

10. Procédé selon l'une des revendications précédentes, dans lequel le système de sorption et le composant réactif acide sont réunis à l'étape c) à l'aide d'un tube à chambres multiples.

11. Dispositif de fabrication d'une préparation de principes actifs, pouvant être obtenue selon le procédé selon la revendication 1 ou l'une des revendications dépendantes de celle-ci, dans lequel le dispositif est composé d'un sac dans lequel deux chambres sont séparées l'une de l'autre par un élément de scellement, dans lequel la première chambre contient le système de sorption basique minéral et l'autre chambre contient le composant réactif acide composé d'acides organiques solides, dans lequel l'élément de scellement est formé pour s'ouvrir en cas d'application d'une pression sur l'une des chambres et mettre ainsi en contact l'un avec l'autre le système de sorption basique minéral et le composant réactif acide composé d'acides organiques solides.

12. Utilisation d'une dispersion de principes actifs produite selon le procédé selon l'une des revendications 1 à 7 comme complément alimentaire ou boisson rafraichissante.

13. Utilisation d'une dispersion de principes actifs produite selon le procédé selon l'une des revendications 1 à 7 comme additif pour des préparations pharmaceutiques.

14. Utilisation d'une dispersion de principes actifs produite selon le procédé selon l'une des revendications 1 à 7 comme additif pour des préparations cosmétiques.

15. Utilisation selon la revendication 14, dans laquelle la préparation cosmétique est une crème.

16. Utilisation d'une dispersion de principes actifs produite selon le procédé selon l'une des revendications 1 à 7 comme additif pour un dentifrice.
